# EUROPÄISCHE PATENTSCHRIFT

(11) **EP 1 062 980 B1**
(45) Veröffentlichungstag und Bekanntmachung des Hinweises auf die Patenterteilung: **01.03.2006**
(21) Anmeldenummer: 00250197.1
(22) Anmeldetag: 23.06.2000
(51) Int. Cl.: A61N 1/372

(54) **Verfahren und Vorrichtung zur Datenübertragung zwischen einem elektromedizinischen Implantat und einem externen Gerät**
Method and device for transmitting data between an electromedical implant and an external apparatus
Procédé et dispositif de transmission de données entre un implant électromédical et un appareil externe

(30) Priorität: 25.06.1999 DE 19930263
(43) Veröffentlichungstag der Anmeldung: 27.12.2000
(73) Patentinhaber: Biotronik GmbH & Co. KG, 12359 Berlin (DE)
(72) Erfinder: Kraus, Michael, 91301 Forchheim (DE); Lang, Martin, 91901 Grossenseebach (DE); Lang, Bernhard, 90537 Feucht (DE); Neudecker, Johannes, 91054 Erlangen (DE); Beetz, Klemens, 91054 Erlangen (DE); Nagelschmidt, Axel, 91052 Erlangen (DE); Potschadtke, Jens, 91052 Erlangen (DE)
(74) Vertreter: Lindner-Vogt, Karin L.

(56) Entgegenhaltungen:
- US-A- 5 562 713

## Beschreibung

Die Erfindung betrifft ein Verfahren und eine Vorrichtung zur Datenübetragung zwischen einem elektromedizinischen Implantat und einem externen Gerät gemäß dem Oberbegriff des Anspruchs 1 beziehungsweise gemäß dem Oberbegriff des Anspruchs 19.

Um den Betrieb eines elektromedizinischen Implantats bzw. den Zustand des betreffenden Patienten zu überwachen, ist es erforderlich, solche Implantate mit Telemetrieeinrichtungen auszustatten. Diese ermöglichen es, im Implantat zur Verfügung stehenden, den Zustand des Patienten und des Implantats widerspiegelnden Daten berührungsfrei in ein externes Gerät auszulesen. Bei solchen elektromedizinischen Implantaten handelt essich beispielsweise um Herzschrittmacher, Defibrillatoren, Kardioverter oder andere elektronisch betätigte bzw, gesteuerte Implantate.

So ist aus der US-Patentschrift US 5,720,770 beispielsweise ein Verfahren zur Datenübertragung zwischen einem elektromedizinischen Implantat mit einer ersten Sender/Empfängereinheit und einem zugeordneten externen Gerät mit einer zweiten Sender/Empfängereinheit, bei dem die Datenübertragung mit einem von der Sende/Empfängereinheit des externen Geräts an die Sender/Empfängereinheit des Implantats gesendeten Auslösesignal beginnt. Dieses Auslösesignal wird im Normalbetrieb des Implantats in vorgebbaren ersten Zeitintervallen gesendet. Bei einer solchen bidirektionalen Funkstrecke, wie sie hier zum Einsatz kommt, sind die Empfänger an beiden Enden der Kommunikationsstrecke stets eingeschaltet um eine Kommunikationsaufnahme des anderen Partners zu detektieren.

Die Energieressourcen innerhalb eines solchen elektromedizinischen Implantats sind jedoch sehr begrenzt, so daß es wünschenswert wäre, zur Erhöhung der Lebensdauer einen möglichst geringen Energieverbrauch zu erzielen.

In der US 5,720,770 wird hierzu vorgeschlagen, die Prozessoren, welche die Telemetrieeinrichtung des Implantats steuern, während der inaktiven Zeiten der Telemetrieeinrichtung auf Minimalbetrieb herunterzufahren und sie bei Eintreffen eines Auslösesignals vom externen Gerät "aufzuwecken" und in den Normalbetrieb hochzufahren, in dem mehr Energie verbraucht wird.

Dieses Verfahren weist jedoch nach wie vor den Nachteil auf, daß der Empfänger des Implantats durchgehend in Betrieb sein muß, um das Auslösesignal des externen Geräts zu erfassen.

Aus der US 5, 562, 713 ist ein Telemetriesystem für die Kommunikation zwischen Implantat und externen Gerät bekannt, bei dem das Implantat auf einer vorbestimmten Park-Frequenz periodisch auf ein Startkommando des externen Gerätes wartet, mit dem die Kommunikation eingeleitet wird. Obwohl bei diesem Verfahren der Empfänger des Implantates nicht nötwendigerweise durch gehend eingeschaltet bleibt, wird die Batterie des Implantates durch das regelmäßige Anschalten seines Empfängers auch dann belastet, wenn aus Sicht des Implantates keine Notwendigkeit für einen Datenaustausch besteht.

Der Erfindung liegt deshalb die Aufgabe zugrunde, ein gattungsgemäßes Verfahren zur Verfügung zu stellen, das einen energiesparenderen Betrieb des Implantats ermöglicht.

Die Aufgabe wird, ausgehend von einem Verfahren gemäß dem Oberbegriff des Anspruchs 1, durch die im kennzeichnenden Teil des Anspruchs 1 angegebenen Merkmale gelöst.

Die Erfindung schließt die technische Lehre ein, daß man einen besonders energiesparenden Betrieb des Implantats erzielt, wenn das Auslösesignal stets von der ersten, implantierten Sender/Empfängereinheit, d.h. der Sender/Empfängereinheit des Implantats ausgesendet wird, wobei wenigstens die Empfangsbereitschaft der ersten Sender/Empfängereinheit nach Aussenden des Auslösesignals für ein zweites Zeitintervall aufrechterhalten wird. Die Empfangsbereitschaft der ersten Sender/Empfängereinheit des Implantats wird nur für einen gewissen Zeitraum aufrechterhalten, der kürzer ist als der Zeitraum bis zum nächsten Auslösesignal, so daß zumindest der Empfängerteil, vorzugsweise auch der Senderteil, der ersten Sender/Empfängereinheit vor dem nächsten Auslösesignal ausgeschaltet wird und somit keine Energie verbraucht. Je nachdem, wie das Verhältnis zwischen Empfangs- und Ruhephasen ausfällt, läßt sich hierdurch eine beträchtliche Energieeinsparung erzielen.

Zudem sendet die zweite, externe Sende/Empfängereinheit auf eine Übertragung von Daten durch die erste, implantierte Sende/Empfängereinheit hin eine erste Quittung an die erste Sende/Empfängereinheit. Hierbei enthält die erste Quittung wenigstens eine erste Steuerinformation zur Steuerung der Empfangsbereitschaft der ersten Sende/Empfängereinheit. So ist es möglich, die Empfangsbereitschaft der ersten Sende/Empfängereinheit abhängig davon zu steuern, ob noch eine Sendung durch die zweite Sende/Empfängereinheit erfolgen soll. Es versteht sich, daß dabei dieEmpfangsbereitschaft, also das zweite Zeitintervall wenigstens so lange andauern muß, daß die erste Quittung noch von der ersten Sende/Empfängereinheit empfangen wird bzw. sich das zweite Zeitintervall zumindest danach bemißt, in welcher Zeit eine solche Quittung zu erwarten ist.

Die Energieeinsparung kann dabei unabhängig vom konkreten Aufbau des Empfängers und Senders durch das erfindungsgemäße Verfahren, d.h. das erfindungsgemäße Kommunikationsprotokoll, erreicht werden.

Bei besonders einfachen Varianten ist das Auslösesignal von einem zu übertragenden ersten Datensatz gebildet oder enthält diesen.

Weiterhin ist es möglich, daß die Empfangsphase durch die erste Quittung oder eine dieser folgende Übertragung der zweiten Sende/Empfängereinheit verändert wird. So kann die Empfangsphase, daß heißt, das zweite Zeitintervall, verkürzt werden, wenn im Rahmen des laufenden Übertragungszyklus keine weitere Sendung der zweiten Sende/Empfängereinheit mehr erfolgen soll und die Empfangsbereitschaft somit sofort bzw. umgehend beendet werden kann. Es ist jedoch auch möglich das zweite Zeitintervall zu verlängern, wenn noch wenigstens eine weitere Übertragung folgen soll.

Vorzugsweise ist auch die erste Quittung von einem zu übertragenden zweiten Datensatz gebildet oder enthält diesen.

Bei bevorzugten Varianten des Verfahrens führt das externe Gerät eine erste Plausibilitätsprüfung der von der ersten Sende/Empfängereinheit übertragenen Daten durch. Hierbei können bestimmte Kontroll-Bits der gesendeten Datensätze nach bestimmten Kriterien überprüft werden. Die erste Quittung enthält dann in Abhängigkeit von der Plausibilität der übertragenen Daten eine zweite Steuerinformation zur Steuerung der ersten Sende/Empfängereinheit. Bei mangelnder Plausibilität der übertragenen Daten enthält die zweite Steuerinformation ein erstes Steuersignal zum Auslösen einer erneuten Übertragung von Daten durch die erste Sende/Empfängereinheit. Hierdurch wird in vorteilhafter Weise eine Erhöhung der Übertragungssicherheit erzielt, da wahrscheinlich fehlerhaft übertragene Daten erneut angefordert werden.

Vorzugsweise führt die erste Sende/Empfängereinheit auf das erste Steuersignal eine erneute Übertragung von Daten durch, sofern eine zur Vermeidung einer Überbeanspruchung der Energieversorgung des Implantats ausreichend geringe Anzahl erneuter Übertragungen nicht überschritten ist. Die Anzahl erneuter Übertragungen ist dabei entweder vorgebbar, insbesondere durch eine entsprechendes Steuersignal des externen Geräts. Alternativ oder zusätzlich kann sie in Abhängigkeit vom Ladezustand des Energiespeichers des Implantats festgelegt sein.

Eine weitere Erhöhung der Wahrscheinlichkeit der Datenauthentizität wird bei bevorzugten Weiterbildungen dadurch erzielt, daß die zweite Sende/Empfängereinheit zur Kontrolle der Übertragung der Daten durch das Implantat bei vorhandener Plausibilität der übertragenen Daten wenigstens einen Teil der übertragenen Daten an die erste Sende/Empfängereinheit zurücksendet. Stimmen diese nicht mit den gesendeten Daten überein, ist die Wahrscheinlichkeit hoch, daß auch schon bei der Übertragung an die zweite Sende/Empfängereinheit ein Fehler aufgetreten ist.

Vorzugsweise sendet das Implantat nach der Kontrolle der Übertragung der Daten über die erste Sende/Empfängereinheit eine zweite Quittung an die zweite Sende/Empfängereinheit. Diese zweite Quittung enthält bei Feststellung einer erfolgreichen Übertragung der Daten eine die Gültigkeit der Übertragung repräsentierende erste Signatur. Im Anschluß an diese Sendung stellt das Implantat wenigstens die Empfangsbereitschaft der ersten Sende/Empfängereinheit, vorzugsweise auch die Sendebereitschaft, ab. Die erneute Abgabe eines Auslösesignals markiert dann den Ablauf der Ruhephase, die hierdurch insoweit hinsichtlich ihrer Dauer optimiert ist.

Weiter vorzugsweise führt das externe Gerät zur weiteren Erhöhung der Wahrscheinlichkeit der Datenauthentizität eine zweite Plausibilitätsprüfung der zweiten Quittung durch. Bei festgestellter mangelnder Plausibilität der zweiten Quittung wird nach Ablauf eines weiteren Zeitintervalls nach Absendung der zweiten Quittung eine erneute Abfrage des Implantats durchführt. Dabei nimmt das Implantat nach Ablauf des weiteren Zeitintervalls die Empfangs- und Sendebereitschaft der ersten Sende/Empfängereinheit für ein erneutes weiteres Zeitintervall auf, das ausreicht, um eine Anfrage vom externen Gerät aufzunehmen und zu beantworten.

Weiterhin erfolgt dabei die Beantwortung der Anfrage durch erneutes Senden der zweiten Quittung und/oder der zuletzt gesandten Daten. Hierdurch ist sichergestellt, daß entsprechend kurze vorübergehende Störungen geringeren Einfluß haben. Zudem kann anhand eines Vergleichs der im externen Gerät dann doppelt vorliegenden zweiten Quittung und/oder der zuletzt gesandten Daten entschieden werde, ob der Datensatz als gültig angenommen werden kann.

Bevorzugt erfolgt auch hier bei Feststellung einer fehlerhaften Übertragung der Daten eine erneute Übertragung von Daten durch die erste Sende/Empfängereinheit, sofern eine zur Vermeidung einer Überbeanspruchung der Energieversorgung des Implantats ausreichend geringe Anzahl erneuter Übertragungen nicht überschritten ist.

Bei günstigen Varianten des erfindungsgemäßen Verfahrens erfolgt die erneute Übertragung nach Ablauf eines Wartezeitintervalls, wobei bei mehrfacher erneuter Übertragung die Länge des Wartezeitintervalls vorzugsweise ansteigt. Hierdurch können systematische Fehler ausgeglichen werden, beispielsweise die vorübergehende Anwesenheit eines Störers, der die fehlerfreie Übertragung unmöglich macht.

Bevorzugt werden nach erneuter Übertragung von Daten durch die erste Sende/Empfängereinheit die Verfahrensschritte beginnend mit der Plausibilitätsprüfung erneut durchlaufen, um die Wahrscheinlichkeit der Datenauthentizität auch in diesem Fall zu erhöhen.

Bei einer weiteren Ausbaustufe kann auch im externen Endgerät Energie eingespart werden. Hierzu ist bei vorteilhaften Weiterbildungen des erfindungsgemäßen Verfahrens die zweite Sende/Empfängereinheit im Ausgangszustand bis zum ersten Datenaustausch mit dem Implantat im wesentlichen permanent empfangsbereit. Wenigstens während des ersten Datenaustauschs wird die Sende- bzw. Empfangsbereitschaft der zweiten Sende/Empfängereinheit auf ein periodisches Sende- bzw.

Empfangsbereitschaftsintervall reduziert, wobei die zweite Sende/Empfängereinheit mit der ersten Sende/Empfängereinheit derart synchronisiert wird, daß sich die Sende- bzw. Empfangsbereitschaftsintervalle der ersten und zweiten Sende/Empfängereinheit wenigstens überschneiden. Durch die Ausschaltzeiten der zweite Sende/Empfängereinheit läßt sich auch hier eine erhebliche Energieeinsparung erzielen, die gerade für extern Geräte, die als tragbares Patientengerät ausgebildet sind, von Vorteil ist.

Vorzugsweise ist dabei vorgesehen, daß im Falle des Nichteintreffens von Übertragungen der ersten Sende/Empfängereinheit bei der zweiten Sende/Empfängereinheit über eine vorgegebene Anzahl von Sende- bzw. Empfangsbereitschaftsintervallen der zweiten Sende/Empfängereinheit das Sende- bzw. Empfangsbereitschaftsintervall der zweiten Sende /Empfängereinheit zum Auffangen eines Auseinanderdriftens der Synchronität verlängert wird.

Bei günstigen Ausführungen des erfindungsgemäßen Verfahrens istwenigstens die Ruhephase während des Betriebs durch Übersenden einer zweiten Steuerinformation durch die zweite Sende/Empfängereinheit an die empfangsbereite erste Sende/Empfängereinheit veränderbar. Hierdurch kann beispielsweise auf eine Veränderung des Patientenzustandes eingegangen werden, indem bei einer Besserung die Übertragungsintervalle verlängert werden, während sie bei einer Verschlechterung seines Zustandes verkürzt werden.

Die Übertragungshäufigkeit ist hierbei entweder später bei bidirektionaler Übertragung durch eine mit dem externen Gerät in Verbindung stehende Servicezentrale steuerbar, oder sie ist vorher durch den Arzt mit Hilfe des Programmers einstellbar. Dadurch kann die Überwachung genau auf den Zustand des Patienten abgestimmt werden.

Bei weiteren günstigen Ausführungen wird die Ruhephase in Abhängigkeit von den Betriebsparametern des Implantats verändert. Diese Betriebsparameter spiegeln gerade auch den Zustand des Patienten wider, so daß sich die Übertragungshäufigkeit des Implantats automatisch an dessen Zustand anpaßt.

Vorzugsweise sendet die erste Sende/Empfängereinheit bei Vorliegen entsprechender Betriebsparameter des Implantats zur Auslösung eines Alarmsignals ein Notfall-Auslösesignal an die zweite Sende/Empfängereinheit aus. Hierdurch kann dann beispielsweise durch eine mit dem externen Gerät in Verbindung stehende Notfallzentrale dann entsprechende Gegenmaßnahmen einleiten.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
- Figur 1: ein Blockschaltbild eines bevorzugten Ausführungsbeispiels einer Vorrichtung zur Durchführung des erfindungsgemäßen Verfahrens;
- Figur 2: einen Ablaufplan des erfindungsgemäßen Verfahrens;
- Figur 3: ein Detail aus Figur 2;
- Figur 4: ein Blockschaltbild einer Variante eines Patientenüberwachungssystems unter Verwendung der vorliegenden Erfindung;
- Figur 5: ein Blockschaltbild einer Variante eines Patientenüberwachungs- und nachsorgesystems unter Verwendung der vorliegenden Erfindung;
- Figur 6: ein Blockschaltbild einer weiteren Variante eines Patientenüberwachungs- und nachsorgesystems unter Verwendung der vorliegenden Erfindung;
- Figur 7: ein Blockschaltbild einerTelemetrieeinrichtung eines elektromedizinischen Implantats unter Verwendung der vorliegenden Erfindung;
- Figur 8: die Sender/Empfängereinheit einer Variante der Ausführung aus Figur 1 ;
- Figur 9: ein Schaltbild eines Senders einer Variante der Ausführung aus Figur 1;
- Figur 10: ein Blockschaltbild einer Variante der Ausführung aus Figur 1.

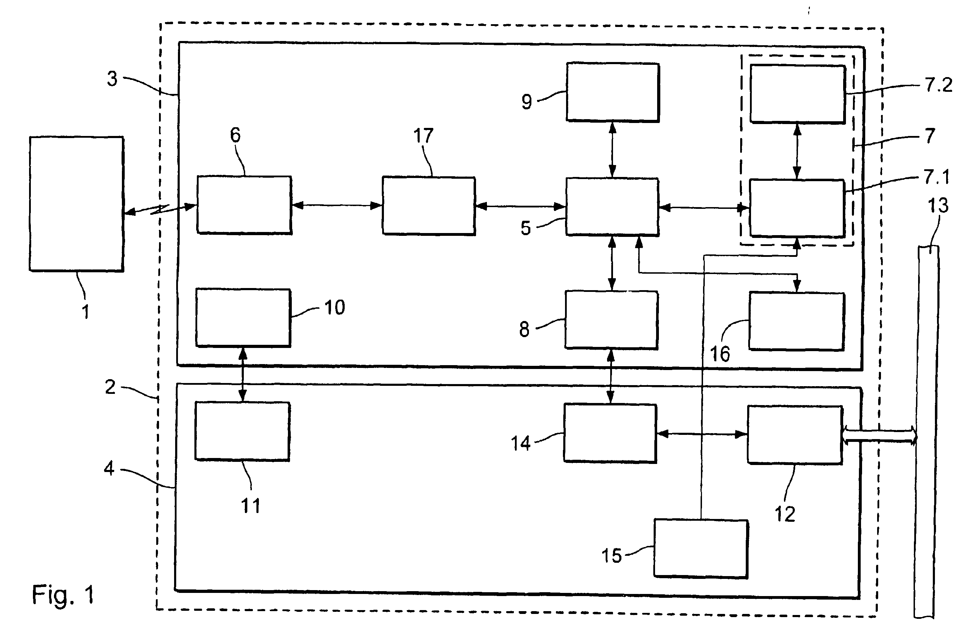

Figur 1 zeigt eine Vorrichtung zur Durchführung des erfindungsgemäßen Verfahrens mit einem elektromedizinischen Implantat 1, das mit einer Telemetrieeinrichtung versehen ist und einem externen Gerät 2, das aus einem Mobilteil 3 und einer Basisstation 4 besteht.

Das Mobilteil 3 weist dabei eine Steuereinrichtung 5 sowie eine erste Schnittstelleneinrichtung 6, eine Mobilfunkeinrichtung 7, eine zweite Schnittstelleneinrichtung 8 und ein Man-Machine-Interface 9 auf, die jeweils mit der Steuereinrichtung 5 verbunden sind.

Die erste Schnittstelleneinrichtung 6 besteht dabei aus einer Telemetrie-Sender/Empfängereinheit, so daß das Mobilteil 3 über eine bidirektionale Telemetriestrecke Kontakt zum Implantat aufnehmen kann. Der Empfänger und der Sender arbeiten im UHF-Bereich bei 403,55 MHz (Bereich 402-405 MHz).

Die Mobilfunkeinrichtung 7 kann erste Daten, die über die erste Schnittstelleneinrichtung 6 aus dem Implantat ausgelesen wurden in Form von SMS-Nachrichten versenden und von einer als Überwachungseinrichtung oder zentraler Speichereinrichtung dienender Servicezentrale angerufen werden.

Die Stromversorgung des Mobilteils erfolgt über Li-Ionenakkus, die im Stromversorgungsteil 10 untergebracht sind.

Die Basisstation 4 besteht aus einem Ladegerät 11 für die Akkus im Stromversorgungsteil 10 und einem als Datenübertragungseinrichtung wirkenden Modem 12 für das Telekommunikationsfestnetz 13, das vom Mobilteil 3 über eine mit der zweiten Schnittstelleneinrichtung 8 zusammenwirkende dritte Schnittstelleneinrichtung 14 angesprochen wird. Die zweite und dritte Schnittstelleneinrichtung 8 und 14 sind dabei als Infrarotschnittstellen (IrDA) ausgebildet.

Das Mobilteil 3 wird zur Ladung und zur Datenübertragung über das Telekommunikationsfestnetz 1 3 in die Basisstation eingesteckt. Die Steuereinrichtung 5 ist dabei so ausgebildet, daß bei Verbindung mit dem Telekommunikationsfestnetz 13 über die zweite Schnittstelleneinrichtung 14 erst nach Ausführung einer vorgegebenen Anzahl erfolgloser Übertragungsversuche über die Mobilfunkeinrichtung 7 die Übertragung der ersten Daten an die Überwachungseinrichtung über das Telekommunikationsfestnetz 13 erfolgt.

Die Basisstation 4 umfaßt weiterhin eine Zusatzantenne 15, die bei Verbindung mit dem Mobilteil 3 mit der Mobilfunkeinrichtung 7 verbunden ist und so die Übertragungsqualität verbessert. Es sind jeweils geeignete λ/4-Antennen vorgesehen, die als Loop- oder Helix-Antennen ausgeführt sind. Die Zusatzantenne ist als Diversity-Antenne 15 ausgeführt.

Das Mobilteil weist eine vierte Schnittstelleneinrichtung 16 auf, die ebenfalls als IrDA-Schnittstelle ausgebildet ist. Diese vierte Schnittstelleneinrichtung 16 wird für die Aufnahme von externen Daten oder für den Service verwendet.

Der Empfänger und der Sender der Sende/Empfangseinheit 6 sind in einem IC integriert. Beide Schaltungsteile bilden eine funktionale Einheit. Als Baudratenstufen sind 4, 8, 16 und 32 KBit/s vorgesehen.

Für das Mobilteil 3 wird nur ein Prozessor als Steuereinheit 5 eingesetzt, der sowohl die Aufgaben der Zentraleinheit, als auch die der Kommunikationseinheit übernimmt. Die Software ist über die IrDA-Schnittstelle 16 in ein Flash-ROM downloadbar. Die Speicherkapazität des internen Flash-ROMs betragt 128 KByte, die des integrierten EEPROMS 4 KByte und die des statischen, externen SRAMs 64 KByte. Die Programmspeichergröße ist somit auf die Kapazität des eingebauten Flash-ROMs begrenzt und kann nicht erweitert werden (Harvard-Struktur). Das externe statische SRAM ist erweiterbar.

Für die SMS-Übertragung wird ein 128 Bit DES Verschlüsselungsverfahren angewendet, das auf einem private-public key System basiert. Die Verschlüsselung wird softwaremäßig realisiert, d.h. es wird kein Verschlüssetungs-IC verwendet. Für einen sicheren Betrieb ist eine Watchdog-Schaltung vorgesehen, die in regelmäßigem Abstand den Prozessor 5 überprüft und ihn wieder in einen sicheren Status überführt, wenn die Programmausführung nicht korrekt abläuft.

Auf dem Weg vom Implantat 1 zum externen Gerät 2 ist wegen der kurzen Reichweite zusätzlich zu der implantatspezifischen Kodierung der Daten keine weitere Verschlüsselung mehr notwendig. Das externe Gerät 2 meldet sich in sechs Fällen sofort über SMS-Nachrichten, ersatzweise über Modem:
- Ein gefährlicher Zustand wird vom Schrittmacher gemeldet.
- Die Übertragungsstrecke zum Schrittmacher funktioniert nicht.
- Ein Selbsttest des externen Geräts meldet einen Fehler.
- Bei Meldung eines Low-Battery-Zustandes vor dem Abschalten.
- Bei Patientenalarm, d.h. der Patient lost durch Drücken einer Taste des Interfaces 9 Alarm aus.
- Auf Anforderung durch die Servicezentrale.

Das externe Gerät 2 führt einen Selbsttest durch und meldet das Ergebnis via SMS an die Servicezentrale, wenn ein Fehler im Betrieb erkannt wird, eine Anfrage der Servicezentrale den Selbsttest des externen Geräts 2 startet oder das externe Gerät 2 nach einem Power-On-Reset wieder ans Netz geht.

Das externe Gerät 2 meldet also selbsttätig Fehler, läßt sich von der Servicezentrale aus prüfen und meldet sich zurück, wenn es wieder betriebsbereit ist (z.B. nach Wiederaufladen der entladenen Akkus). Der SMS-String für den Selbsttest wird nur übertragen, wenn obige drei Vorbedingungen dies erfordern. Im normalen Übertragungsstring ist keine Information zum Selbsttest enthalten. Dieser Selbsttest ist dazu da, Rückschlüsse auf die Funktion der Hardware zuzulassen. Es kann dann im Servicezentrum entschieden werden, wie diese Fehler behandelt werden müssen. Als Möglichkeiten der Selbstdiagnose des externen Gerätes können folgende Kriterien herangezogen werden:
a) Die Prüfsumme über den Programmcode (Flash-ROM-, EEPROM-, ROM-Fehler, Programm fehlerhaft geladen) ergab einen Speicherfehler.
b) Ein Speichertest des RAM ergab einen Speicherfehler.
c) Ein temporärer Ausfall des Mobilfunknetzes ist aufgetreten (kann auch Hardwarefehler bedeuten; das Auslesen der Mobilfunkeinrichtung 7 kann hier Klarheit schaffen).
d) Ein Timeout bei der Kommunikation mit der Servicezentrale ist aufgetreten (wie c).
e) Ein empfangener Datenstring ist zu kurz oder Prüfsummenfehler ist aufgetreten (Hardwarefehler in der Telemetrie oder Empfangsstörung bzw. Reichweiteprobleme).

Für die Mobilfunkeinrichtung 7 ist vorgesehen, daß über SMS die Feldstärkeinformation mit den dazugehörigen Nummern der current and adjoined cells und die Bitfehlerrate übertragen wird. Anhand dieser Ortungsinformationen ist eine Ortung des externen Geräts 2 in Notfällen, in denen der Patient handlungsunfähig ist, möglich.

Die Mobilfunkeinrichtung 7 um faßt ein Mobilfunkmodul 7.1 und einen als SIM-Karte ausgebildeten Speicher 7.1. Der SIM-Kartenleser kann unter dem Mobilfunkmodul 7.1 direkt in die Leiterplatte gelötet werden. Dem Anwender ist damit die SIM-Karte 7.1 nicht zugänglich. Im Speicher der SIM-Karte 7.1 wird bzw. werden zusätzlich die Rufnummer(n) der Servicezentrale permanent abgelegt. Via SMS kann die Nummer der Servicezentrale auf der SIM-Karte geändert werden. Die alte Nummer wird auf der SIM-Karte zur Sicherheit auf einem anderen Speicherplatz gehalten und benutzt, wenn das externe Gerät 2 mit der neuen Nummer keine Verbindung aufbauen kann. Zusätzlich verwendet das Mobilfunkmodul 7.1 die SIM-Karte zur Zwischenspeicherung von SMS-Nachrichten.

Die Stromversorgung des Mobilteils 3 ist mit Li-lonen-Akkus realisiert. Sie können das Mobilteil 3 im voll aufgeladenen Zustand mindestens 20h (Standbyzeit + 16 SMS-Übertragungen) ohne Verbindung mit der Basisstation 4 versorgen. Bei eingeschaltetem Mobilteil 3 sind die Akkus nach ca. 4h in der Basisstation wieder voll aufgeladen.

Die Dimensionierung der Akkus gilt für das Lebensdauerende. Es ist deshalb ein 1000 mAh Li-lonen-Akku vorgesehen.

Das Mobilteil 3 wird zum Laden in die Basisstation 4 eingesteckt. Dabei wird die Stromversorgung mit dem Mobilteil 3 verbunden. Das Mobilteil 3 ist wahrend des Ladens in der Basisstation 4 voll funktionsfähig. Die Spannungswandler in der Basisstation sind bis 40 V ausgelegt, so daß ein Anschluß zum Laden aus einem Kfz-Netz möglich ist. Die Steuereinrichtung 5 überwacht die Akkuspannung und die Spannungsversorgung am Mobilfunkmodul 7.1 .

Die zweite Schnittstelleneinrichtung 8 ist nach dem IrDA-Standard auszuführen. Sie arbeitet bidirektional und kann im halb-duplex-Betrieb Daten übertragen. Diese Schnittstelle 8 wird ausschließlich zum Betrieb des Modems 12 in der Basisstation genutzt. Die IR-Schnittstelle 8 des Mobilteils 3 und die IR-Schnittstelle 14 der Basisstation 4 sind dauernd empfangsbereit. Das Mobilteil 3 tauscht mit der Basisstation 4 Daten aus, sobald es in diese eingesteckt ist. Damit ist dem Mobilteil 3 jeweils bekannt, ob ein Modem 12 in der Basisstation 4 bestückt ist.

Da es sich bei den zu übertragenden Nachrichten um sehr kurze Datenblöcke handelt, ist eine Übertragungsgeschwindigkeit 9600 Baud ausreichend. Das Modem 12 ist in der Basisstation 4 integriert und bei Bedarf durch den Servicetechniker bestückbar. Das Modem 12 kann einfach in die Basisstation eingeschnappt werden und wird dann über eine lrDA-Schnittstelle 14 bedient. Das Modem 12 ist auf einen Softwarehandshake umschaltbar, da über die IrDA-Schnittstellen 8 und 14 nur diese Art der "optischen 2-Draht-Übertragung" unterstützt wird.

War die Mobilfunkübertragung erfolglos, d.h. im Mobilteil 3 liegen noch Daten vor, die nicht über das Mobilfunknetz an die Servicezentrale übertragen werden konnten, wird sofort nach dem Einstecken des Mobilteils in die Basisstation 4 das Modem 12 benutzt. Falls also die Mobilfunkübertragung nicht funktioniert oder das externe Gerät 2 in einem Land betrieben wird, das keinen Mobilfunkstandard unterstützt, erfolgt die Meldung über das Modem, sobald das Mobilteil in die Basisstation gesteckt wird.

Die Reichweite der fünften Schnittstelleneinrichtung, die ebenfalls als lrDA-Schnittstelle ausgeführt ist, beträgt wenigstens 0,4m. Die lrDA-Schnittstellen sind möglichst energiesparend ausgelegt.

Die fünfte Schnittstelleneinrichtung 16 ist so angeordnet, daß sie von einem Gerät, das neben dem externen Gerät 2 steht, benutzt werden kann, wenn das Mobilteil 3 in die Basisstation 4 eingelegt ist. Sie arbeitet bidirektional und kann im halb-duplex-Betrieb Daten übertragen. Die Übertragungsgeschwindigkeit der fünften Schnittstelleneinrichtung 16 startet bei 9600 Baud und kann dann bis auf 115 KBaud hochgeschaltet werden.

Mit dieser Schnittstelle 16 kann beispielsweise ein Servicetechniker unter Verwendung z.B. eines Laptops mit IrDA-Schnittstelle mit dem Mobilteil 3 kommunizieren eine andere Möglichkeit ist, daß das externe Gerät als Data-Logger arbeitet, indem Daten (z.B. als Blutzuckermonitor) eingelesen werden, die dann ebenfalls via SMS an die Servicezentrale geschickt werden. Die Reichweite der IrDA-Schnittstelle 16 beträgt mindestens 0,4m.

Die fünfte Schnittstelleneinrichtung 16 ist so anzuordnen, daß die Kommunikation zwischen Basisstation 4 und Mobilteil 3 sicher funktioniert, d.h. Beeinflussungen der zweiten Schnittstelleneinrichtung 8 durch die fünfte Schnittstelleneinrichtung 16 ausgeschlossen sind.

Die serielle Schnittstelle für beide IrDA-Schnittstellen 8 und 1 6 und die Schnittstelle zur Mobilfunkeinrichtung 7 liegen parallel, so daß immer nur eine Einheit betrieben werden kann. Die lrDA-Schnittstellen 8 und 16 sind parallel geschaltet. Beide Schnittstellen sind dauernd empfangsbereit, so daß jederzeit eine externe Anforderung bedient werden kann. Die Software muß dann lediglich noch feststellen, bei weicher IrDA-Schnittstelle die Empfangsanforderung vorliegt.

Das Netzteil 11 in der Basisstation versorgt das Ladegerät für die Li-lonen-Akkus und liefert die Versorgung für die lrDA-Schnittstelle 14 und das Modem 12. Das Ladegerät ist mit einem für Li-lonenakkus geeigneten Regler und einer Überwachungsschaltung ausgerüstet. Es ist für einen maximalen Ladestrom von 350mA ausgelegt. Das Netzteil 1 ist für eine primäre umschaltbare Wechselspannung von 220/110V, 50/60Hz dimensioniert, wobei ein Bereich von 100 bis 240 V für die primäre Eingangsspannung und ein Frequenzbereich von 47-63 Hz bevorzugt ist. Es wird Schutzklasse 2 für das externe Gerät 2 verwendet. Die Stromversorgung in der Basisstation 4 kann auch durch ein geeignetes Steckernetzteil ersetzt werden.

Bei einer nicht dargestellten Ausführung sind Telemetrieeinheit 6 für die Kommunikation mit dem Implantat 1 inklusive einer Sende/Empfangsantenne für die Telemetriefrequenz und einer Ansteuerelektronik sowie einer vierten Schnittstelleneinrichtung 17 in ein separates Gehäuse eingebaut. Dieses separate Gehäuse weist ein mechanisches und elektrisches Interface für das Batteriefach eines Mobiltelefons auf. D. h. der Batteriepack des Mobiltelefons ist entnommen und statt dessen das separate Gehäuse eingesetzt, so daß sich eine mechanisch stabile Einheit aus Mobiltelefon und separatem Gehäuse ergibt, welche gegebenenfalls die bisherige äußere Kontur des Mobiltelefons überragt.

Gleichzeitig wird die elektrische Verbindung zwischen dem Mobiltelefon und der Telemetrieeinheit im separaten Gehäuse hergestellt (zumindest bezüglich der Energieversorgung über die Anschlüsse des Batteriepacks).

Zur Energieversorgung des Gesamtsystems weist das separate Gehäuse ein Batteriefach auf, in welches der originale Batteriepack des Mobiltelefons oder aber eine andere geeignete Batterie oder ein geeignetes Batteriepack eingelegt werden kann. Diese Batterie dient dann zur Energieversorgung des Gesamtsystems aus Mobiltelefon und Telemetrieeinheit und kann über die serienmäßigen Ladeanschlüsse am Mobiltelefon geladen werden.

Zur Datenverbindung zwischen der Telemetrieeinheit und dem Mobiltelefon gibt es drei grundsätzliche Möglichkeiten:
1. über die am Mobiltelefon vorhandene Schnittstelle mit elektromechanischem Stecker,
2. über die am Mobiltelefon vorhandene Schnittstelle.
3. über eine zusätzliche im Batteriefach des Mobiltelefons integrierte Schnittstelle (elektromechanisch oder Infrarot)

Bei der 1. und 2. Variante existiert zwischen dem separaten Gehäuse und der im Mobiltelefon integrierten Schnittstelle eine mechanische oder optische Verbindung, d.h. das separate Gehäuse oder ein fester Fortsatz desselben überdeckt die Schnittstelle des Mobiltelefons und enthält an dieser Stelle eine geeignete Steckverbindung oder reicht bis in die Nahe der Schnittstelle des Mobiltelefons und enthält an diesem Ende eine Infrarot-Schnittstelle. Diese Lösungen bedingen eine spezielle Software für das Mobiltelefon, welche eine Steuerung, die über diese Schnittstelle initiiert wird, zuläßt.

Bei der 3. Variante erfordert die im Batteriefach integrierte Schnittstelle eine mechanische und elektrische Modifikation des Mobiltelefons. Durch eine geeignete Verbindung dieser Schnittstelle mit der Elektronik des Mobiltelefons wird eine direkte Steuerung des Mobiltelefons durch die Telemetrieeinheit möglich.

In beiden Fallen bleibt die bekannte, normale Funktion eines Mobiltelefons erhalten. Gegebenenfalls kann eine Vorrangsteuerung für Telemetrieübertragungen vorgesehen werden. Vorzugsweise kann die Elektronik des Mobiltelefons durch die permanent in Empfangsbereitschaft befindliche oder wahlweise zeitgesteuerte Telemetrieeinheit bei Bedarf automatisch in Betrieb genommen werden (Ereignissteuerung).

Die Basisstation 4 ist mit einer Leuchtdiode ausgestattet, die aufleuchtet, wenn das Mobilteil 3 eingesteckt ist und ein Ladestrom die Akkus auflädt. Die Leuchtdiode erlischt, wenn das Mobilteil 3 entnommen bzw. kein Strom mehr vom Mobilteil 3 aufgenommen wird. Die Anzeige der Leuchtdiode wird für die Dauer der Übertragung, aber mindestens eine Sekunde invertiert, wenn über das eingebaute Modem 12 eine Nachricht verschickt oder empfangen wird. Die Basisstation 4 quittiert das Einlegen des Mobilteils 3 oder das Entnehmen des Mobilteils 3 mit einem kurzen Ton eines Buzzers.

Es ist kein Netzschalter vorgesehen. Will man die Basisstation 4 vom Netz trennen, kann das Steckernetzteil gezogen werden.

Das Mobilteil 3 besitzt einen versenkten Ein/Aus-Schalter, der nicht unbeabsichtigt betätigt werden kann. Die Schalterposition ist beschriftet, so daß ersichtlich ist, ob das Mobilteil 3 eingeschaltet ist. Dieser Schalter ist notwendig, da das Mobilteil 3 mit der Mobilfunkeinrichtung 7 in einigen Umgebungsbereichen ausgeschaltet werden muß. Dies ist z. B. während eines Fluges und in bestimmten Bereichen von Krankenhäusern notwendig.

Ins Mobilteil 3 ist eine Taste für den Patientenalarm eingesetzt. Die Taste ist so auszulegen, daß sie vor unbeabsichtigter Fehlbedienung sicher geschützt ist. Diese Notfalltaste ist deshalb versenkt angeordnet. Zusätzlich kann sie durch einen Deckel geschützt sein, der erst geöffnet werden muß, um den Patientenalarm auszulösen, oder der Fehlbedienungsschutz ist durch eine Zeitsperre in der Software ausgeführt, welche die Tastenfunktion erst als gültig annimmt, wenn die Taste länger (z.B. mehr als 3 s) gedruckt wird. Der Patientenalarm wird sofort über eine SMS an die Servicezentrale weitergeleitet.

Der erfolgreich ausgelöste Patientenalarm wird akustisch durch den Buzzer quittiert. Danach kann die Taste wieder losgelassen werden. Weiterhin ist eine Leuchtdiode für 10s dauereingeschaltet und leuchtet dann jede Minute nochmals für 2s zur Erinnerung an den ausgelösten Patientenalarm. Der Buzzer wird immer gleichzeitig mit der Leuchtdiode betätigt, so daß auch alte Menschen diese Quittung des externen Geräts wahrnehmen können. Der Alarm wird erst gelöscht, wenn die Servicezentrale den Patientenalarm über SMS quittiert und so sichergestellt ist, daß der Alarm in der Servicezentrale angekommen ist.

Durch diese Maßnahmen wird verhindert, daß bei Auslösung des Patientenalarms in einem gefährlichen Zustand des Patienten weitere Maßnahmen unterlassen werden, wenn der Patientenalarm nicht erfolgreich ausgeführt werden konnte. Will man den nicht quittierten Patientenalarm löschen, muß das Mobilteil 3 ausgeschaltet werden. Nach dem Neustart ist das Mobilteil wieder im Normalzustand. Via SMS meldet sich das Mobilteil automatisch in der Servicezentrale zurück. Dort kann dann erkannt werden, daß das externe Gerät zurückgesetzt wurde und der Patientenalarm gelöscht ist.

Sind 10% der maximalen Akkuladung erreicht, wird durch eine blinkende Leuchtdiode der Low-Battery-Zustand dargestellt. Das Mobilteil 3 hat dann noch minimal etwa 2 Stunden Gangreserve. Da das Mobilteil 3 für den Patienten nicht immer sichtbar ist, zeigt der Buzzer durch einen kurzen Alarm das Eintreten des Low-Battery-Zustandes an. Der Buzzer sollte etwa alle 5 Minuten wieder Alarm auslösen, um den Patienten an das Aufladen des Akkus zu erinnern. Nach dem Wiederaufladen des Akkus wird die akustische Alarmfunktion des Low-Battery-Zustandes wieder scharf gestellt. Der Buzzer muß auf einer Frequenz von etwa 2 kHz und darunter arbeiten und entsprechend laut sein, so daß auch ältere Patienten den Alarmton sicher hören können.

Bei Auftreten des Patientenalarms wird die Leuchtdiode und der Buzzer für die Quittierung des Patientenalarmes verwendet. Die erfolgreiche Durchführung des Patientenalarms hat gegenüber der Low-Battery-Anzeige eine höhere Priorität. Die Low-Battery-Anzeige wird erst wieder angezeigt, wenn das Servicezentrum den Patientenalarm quittiert hat. Soll eine Störung der Umgebung durch den Buzzer verhindert werden, ist das Mobilteil 3 auszuschalten.

Das externe Gerät 2 wird mit einer SIM-Karte ausgeliefert, die im Rufnummernspeicher als Voreinstellung die Nummer einer Servicezentrale enthält. Es können mehrere Servicezentralen betrieben werden. Dies macht besonders Sinn, wenn dadurch vermieden wird, daß die SMS-Nachricht über Grenzen geschickt werden muß. Es ist daher eine Vorgehensweise vorgesehen, wie Patientengeräte den Servicezentralen zugeordnet und auch an weitere Servicezentralen überwiesen werden können, ohne daß ein Servicetechniker zu dem Patientengerät fahren muß, um es umzustellen.

Der Patient meldet sich bei einer Servicezentrale an. Dadurch ist der Servicezentrale die Adresse des Kunden bekannt und es kann entschieden werden, welche Servicezentrale den Dienst übernimmt. Schaltet der Kunde sein externes Gerät ein, dann meldet es sich bei der voreingestellten Servicezentrale an, indem es sofort eine SMS an diese abschickt. Die Servicezentrale kann nun durch Rücksendung einer speziellen SMS die Rufnummer übertragen, die in Zukunft verwendet werden soll. Das Patientengerät wird so nachträglich einer anderen Servicezentrale zugeordnet.

Die erste Rufnummer wird auf der SIM-Karte zur Sicherheit auf einem Speicherplatz gehalten und auch benutzt, wenn das externe Gerät 2 mit der neuen Nummer keine Verbindung aufbauen kann. Auf diese Weise wird sichergestellt, daß der Kontakt stets erhalten bleibt. Die Datenbanken der Servicezentralen gleichen sich untereinander ab, so daß stets klar ist, welches Implantat bzw. externe Gerät von weicher Servicezentrale bedient wird. In der Datenbank sind entsprechende Felder vorzusehen.

Die Rufnummernverwaltung (Schreiben und Lesen der Nummern auf und von der SIM-Karte) muß vom Zentralprozessor des externen Geräts übernommen werden. Im Betrieb kann nur eine weitere (zweite) Nummer angenommen werden. Diese wird verwendet, wenn der Kontakt zu der "neuen Servicezentrale" erfolgreich hergestellt werden kann. Ist dies nicht der Fall, wird wieder die "alte Rufnummer verwendet". Wird das externe Gerät erneut an eine weitere Servicezentrale überwiesen, wird die zweite Nummer überschrieben, so daß der Ablauf immer so ist, daß zunächst die zweite Nummer versucht wird und bei einem oder mehreren Fehlversuch(en) die erste Nummer benutzt wird. Der Vorteil dieses Verfahrens liegt darin, daß eine volle Flexibilität möglich ist, die sichere Rufnummer aber gespeichert bleibt, so daß ein völliger Zusammenbruch der Verbindung sicher vermieden wird.

In der SIM-Karte wird eine Art LiFo-Speicher für eine maximale Speichertiefe (z. B. zehn Rufnummern) organisiert. Gibt die bisher zuständigen Servicezentrale eine neue Rufnummer vor, wird diese als aktuellste Nummer verwendet. Entstehen aber mit dieser neuen Rufnummer Übertragungsprobleme, wird jeweils die nächst ältere Nummer verwendet, bis schließlich die voreingestellte Nummer an der Reihe ist. Ist der Speicher belegt, z.B. mit 9 Nummern und der voreingestellten Nummer, wird die älteste, also die Nummer die der voreingestellten am nächsten liegt gelöscht.

Der Vorteil diese Verfahrens ist seine hohen Redundanz, da anzunehmen ist, daß sicher mit einer der gespeicherten Servicezentralen eine Verbindung zustande kommt. Der Nachteil ist, daß unter Umständen viele Servicezentralen Daten von einem externen Gerät erhalten, die dann erst zusammengestellt und gemeinsam ausgewertet werden können.

Da das externe Gerät 2 mit einem Modem 12 ausgerüstet ist, kann der Rufnummernspeicher in der SIM-Karte noch zusätzlich für diese Rufnummern eingesetzt werden. Die Organisation kann man in Anlehnung an die Verwaltung der Mobilfunknummern gestalten. Durch das Abspeichern der Rufnummern auf der SIM-Karte, kann bei Austausch des externen Geräts beim Kunden, z.B. bei einem Defekt, die SIM-Karte wieder in das neue Gerät gesteckt werden, so daß die Art des Verbindungsaufbaus zu den Servicezentralen lückenlos übernommen wird.

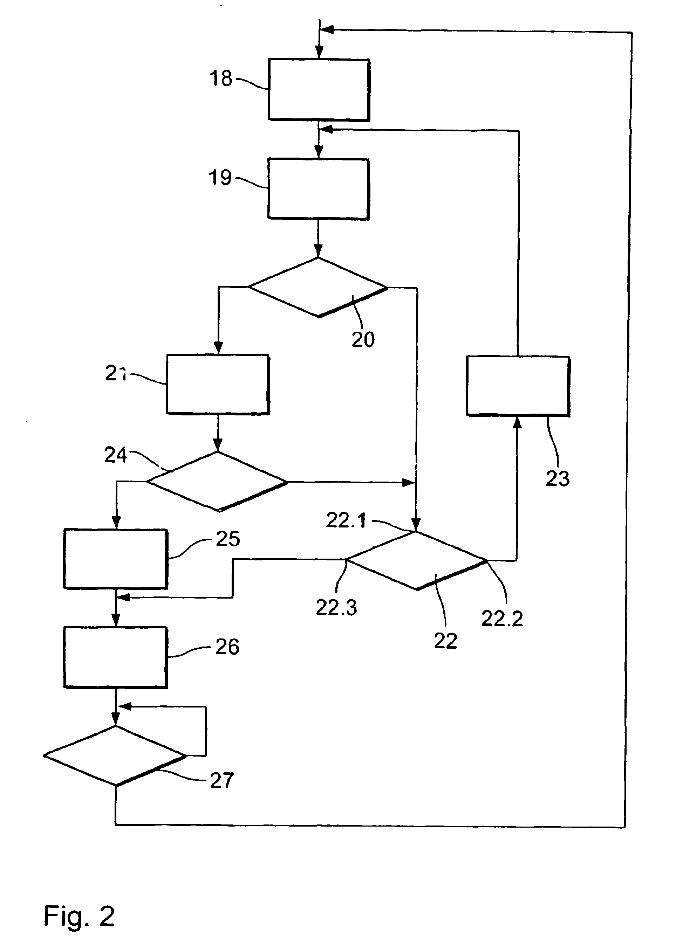

Figur 2 zeigt einen Ablaufplan des erfindungsgemäßen Verfahrens zur Datenübertragung, das zur Datenübertragung zwischen dem Implantat 1 und dem externen Gerät 2 verwendet werden kann.

Die erste Sender/Empfängereinheit des Implantats 1 umfaßt dabei einen Sender (VHF) und einen Empfänger (VHF oder LF). Die zweite Sender/Empfängereinheit des externen Geräts 2 umfaßt einen Empfänger (VHF) und einem Sender (VHF oder LF). Beide kommunizieren bidirektional über eine Funkstrecke. Die Baudrate Implantat externes Gerät beträgt 4, 8 oder 16 kBit/s. Für die Strecke externes Gerät - Schrittmacher wird bei VHF-Übertragung wieder die gleiche Baudrate verwendet, bei LF-Übertragung ca. 10 Bit/s.

Das Auslösesignal zur Datenübertragung wird dabei stets von der ersten Sender/Empfängereinheit des Implantats 1 ausgesandt, wozu in einem Schritt 18 die Sender/Empfängereinheit eingeschaltet wird.

In einem weiteren Schritt 19 erfolgt dann die Übertragung der ersten Daten aus dem Implantat 1 an das externe Gerät 2. Es wird ein String übertragen, der aus 17 Byte besteht. Dies sind 1 Byte Synchronisation, 4 Byte Identifikation, 1 Byte laufende Nummer der Übertragung, 1 Byte Länge des übertragenden Strings, 8 Byte Daten, 2 Byte Security (CRC). Bei asynchroner Übertragung ergibt sich ein Overhead von 3 Bit je Byte, so daß 17*11 Bit = 187 Bit übertragen werden müssen.

In Schritt 20 erfolgt daraufhin eine Plausibilitätsprüfung der übersandten ersten Daten durch das externe Gerät. Hierbei erfolgt beispielsweise eine Kontrolle der CRC-Bits im Bezug auf den gesendeten String oder eine Überprüfung, ob die Synchronisationsbits die vorher bekannten Werte aufweisen. Weiterhin kann überprüft werden, ob die Stringlänge mit der gesendeten Stringlänge übereinstimmt und/oder ob die laufende Nummer der Übertragung korrekt ist.

Eine Überprüfung von Flags entfällt, da im externen Gerät 2 kein Handling der Flags erfolgt. Bei Erweiterungen der Telemetrie kann somit immer der gleiche Typ eines externen Geräts verwendet werden. Eine Überprüfung der Implantatnurnmer entfällt, da das externe Gerät die Information mehrerer Implantate weiterleiten kann.

Nach der erfolgten Plausibilitätsprüfung wird eine erste Quittung an das Implantat 1 gesendet, wobei die erste Quittung eine erste Steuerinformation zur Steuerung der Empfangsbereitschaft der erste Sender/Empfängereinheit enthält.

Falls der Datensatz als gültig erkannt wurde, wird hierbei mit oder in der ersten Quittung in einem Schritt 21 ein Kontrolldatensatz übersandt, der durch das Implantat 1 kontrolliert wird. Hierbei gibt es zwei Möglichkeiten:

Als sicherste Methode wird der zuvor gesandte erste Datensatz komplett an das Implantat 1 zurückgeschickt, das dann den abgeschickten mit dem empfangenen Datensatz vergleicht. Es würden dann erneut 187 Bit zurückgeschickt.

Alternativ werden die Synchronisation (8 Bit), Identifikation (32 Bit), die laufende Nummer der Übertragung (8 Bit) und die CRC-Bits (16 Bit) zur Kontrolle an das Implantat 1 zurückgeschickt, das dann prüft, ob der gesendete String (Identifikation, Nummer der Übertragung) und ob die empfangenen CRC-Bits mit den CRC-Bits des gesendeten Nachricht übereinstimmen. Es werden dann 8 statt 17 Byte wie oben, d.h. es sind 64 Bit + 8 * 3 Bit = 88 Bit übertragen (etwa die Hälfte bezogen auf Variante 1).

Erkennt das externe Gerät im Schritt 20, daß die Daten nicht plausibel sind, werden, sofern in einem Schritt 22 festgestellt wurde, daß eine vorgegebene Anzahl erneuter Übertragungen noch nicht überschritten ist, in einem Schritt 23 gültige Daten vom Schrittmacher angefordert. Diese Anfrage kann aus Synchronisation (8 Bit), Identifikation (32 Bit), der laufenden Nummer der Nachricht (8 Bit) und einem vereinbarten Code (8 Bit) bestehen. Diesen String kann noch mit einem CRC-Byte abgesichert sein. Es werden dann 64 Bit + 8 * 3 Bit = 88 Bit gesendet, um eine Sendeabfrage des Schrittmachers einzuleiten.

Hat das externe Gerät 2 die ersten Daten als plausibel erkannt wurde der String oder die CRC-Bits in Schritt 21 an das Implantat zu Kontrolle in Schritt 24 abgeschickt. Ist die Prüfung erfolgreich sendet das Implantat in Schritt 25 eine zweite Quittung an das externe Gerät 2. Das Implantat 1 stellt dann in Schritt 26 alle Sende- und Empfangsaktivitäten ein, bis ein nach Ablauf des ersten Zeitintervalls neuer Datensatz zur Sendung ansteht. Der Ablauf des ersten Zeitintervalls wird dabei in Schritt überprüft.

Diese zweite Quittung kann aus Synchronisation (8 Bit), Identifikation (32 Bit), der laufenden Nummer der Nachricht (8 Bit) und einem vereinbarten Code (8 Bit) bestehen. Diesen String kann noch mit einem CRC-Byte abgesichert sein. Es werden 64 Bit + 8 * 3 Bit = 88 Bit gesendet.

Nach dem Empfang der zweiten Quittung wird das Patientengerät die nun verifizierten ersten Daten an die Servicezentrale weiterleiten. Der erste Datensatz ist als gültig markiert und die Servicezentrale weiß, daß diese Daten mit einer sehr hohen Wahrscheinlichkeit richtig sind. Das externe Gerät behält diese ersten Daten, bis das Implantat einen neuen, als richtig verifizierten Datensatz abliefert.

Fällt die Kontrolle in Schritt 24 negativ aus, d.h. sind die Kontrolldaten nicht identisch mit den Ausgangsdaten, erfolgt die Sendung des ersten Datensatzes erneut. Das Implantat versucht es dann nach einer festgelegten Zeitspanne nochmals. Das externe Gerät 2 informiert die Servicezentrale über die Anzahl falsch übertragener Datenpakete.

Falls bei der Sendung der zweiten Quittung, die das Implantat an das externe Gerät schickt, ein Fehler auftritt (z.B. andere Implantatnummer, oder Quittungscode stimmt nicht) werden die ersten Daten trotzdem per SMS an die Servicezentrale gesandt, es wird aber zusätzlich vom externen Gerät 2 gemeldet, daß die zweite Quittung des Implantats 1 nicht gültig übertragen werden konnte.

Weiterhin besteht die Möglichkeit, daß das Implantat 1 nach Absendung der zweiten Quittung nach eines genau festgelegten dritten Zeitintervalls wieder auf Empfang geht und eine erneute Anfrage vom externen Gerät 2 beantwortet. Hierbei kann die zweite Quittung oder der erste Datensatz erneut gesendet werden. Das externe Gerät 2 hat dann entweder den ersten Datensatz oder die zweite Quittung doppelt vorliegen und kann nun entscheiden, ob der erste Datensatz als gültig angenommen werden kann.

Für den Fall, daß das externe Gerät 2 erkannt hat, daß die Daten ungültig sind, wird wie erwähnt statt des Strings oder der CRC-Daten eine erste Quittung geschickt, die eine neue Sendung anfordert. Diese erste Quittung könnte wieder aus 88 Bit bestehen. Das Implantat 1 kann sofort, nachdem er geprüft hat, daß er angesprochen ist, mit der erneuten Sendung beginnen, ohne daß erst die empfangenen Daten ausgewertet werden müssen.

Die Sende/Empfangszyklen können sich mehrmals wiederholen. Es muß aber aus Gründen der Energieersparnis darauf geachtet werden, daß nach einer bestimmten Anzahl von Sendungen abgebrochen wird, um den Energiespeicher des Implantats nicht unnötig zu entladen. Das externe Gerät sollte dann trotzdem die Daten an die Servicezentrale senden, die Daten müssen aber als nicht verifiziert markiert sein. Die Daten werden im externen Gerät 2 gehalten, bis ein neuer Datensatz vom Implantat gesendet wird. Zusätzlich sollte der letzte gültige Datensatz im externe Gerät gehalten werden, den dann die Servicezentrale abfragen kann. Bei mehreren Implantaten, die zusammen mit dem externen Gerät 2 arbeiten, sind die jeweils letzten Datensätze jedes Implantats im externen Gerät 2 zu halten.

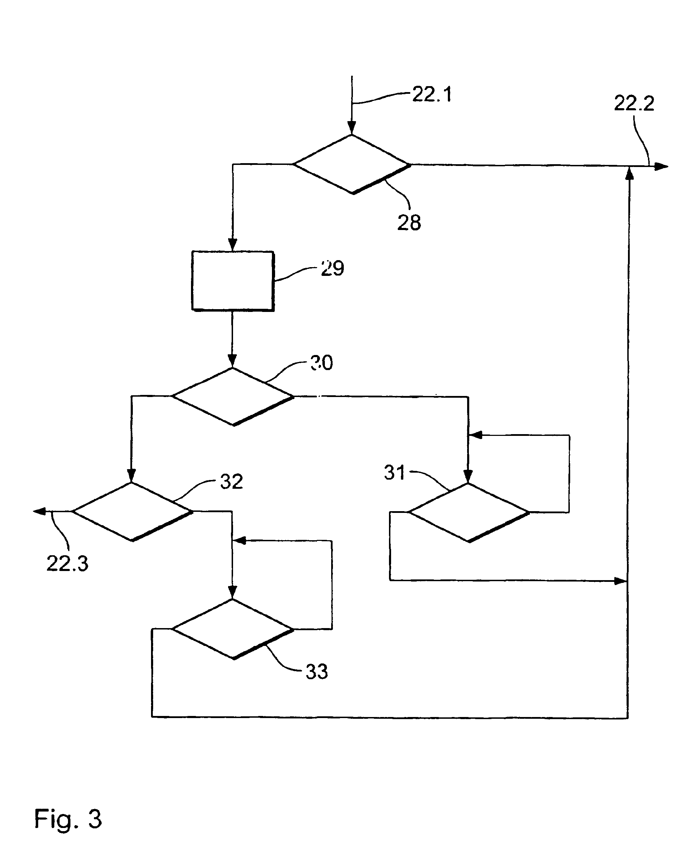

Bei den Wiederholungen der Sendung durch das Implantat 1 kann folgende Strategie eingeschlagen werden, die in Figur 3 als Ablaufdiagramm dargestellt ist. Figur 3 stellt Schritt 22 aus Figur 2 mit dem Eingang 22.1 und den Ausgängen 22.2 und 22.3 im Detail dar.

Zunächst wird ein zweites Mal versucht in möglichst kurzem Abstand die Sendung erfolgreich zu übertragen. Dabei wird in Schritt 28 untersucht, ob ein Zähler Z einen vorgegebenen ersten Wert, hier 2, noch nicht überschritten hat. Der Zähler Z repräsentiert dabei der die Anzahl der durchgeführten Übertragungsversuche. Er wird in Schritt 18 auf Null gesetzt und mit jedem Durchlaufen von Schritt 19 um 1 erhöht. Ist der erste Wert noch nicht überschritten wird sofort über Ausgang 22.2 eine neue Übertragung initiiert.

Ist jetzt keine korrekte Übertragung möglich gewesen, wartet das System merklich, da von einem systematischen Fehler ausgegangen werden kann. Dies kann z.B. ein Störer sein, der für einige Zeit die Übertragung unmöglich macht (z.B. nicht ausreichend entstörtes Elektrogerät). Dies geschieht, nach dem in Schritt 29 ein Timer auf Null gesetzt wurde und in Schritt 30 untersucht wurde, ob der Zähler Z einen vorgegebenen zweiten Wert, hier 3, noch nicht überschritten hat, durch die Warteschleife 31.

Nach einer Wartezeit von z.B. 5 Minuten wird erneut ein Zyklus gestartet. Ist dieser ebenfalls erfolglos, erfolgt die nächste Sendung in einer Stunde. Dies geschieht, nach dem in Schritt 29 der Timer auf Null gesetzt wurde und in Schritt 32 untersucht wurde, ob der Zähler Z einen vorgegebenen dritten Wert, hier 4, noch nicht überschritten hat, durch die Warteschleife 33.

Ist dies erneut erfolglos, wird erst wieder gesendet, wenn neue Daten im Schrittmacher zur Sendung bereitliegen.

Der Empfänger des externen Geräts 2 ist bei bestimmten Ausführungen des Verfahrens jederzeit bereit Daten vom Implantat zu empfangen.

Zu anderen Varianten des Verfahrens ist folgendes anzumerken: In regelmäßigen Abständen werden Sendungen vom Implantat 1 zum externen Gerät 2 geschickt, die entweder Daten enthalten oder nur anzeigen, daß das Implantat 1 aktiv und empfangsbereit ist. Diese Sendungen werden von der externen Gegenstelle empfangen, analysiert und quittiert. Die Quittungen werden in einem festen Zeitfenster nach der Sendung erwartet, so daß der Empfänger im Implantat 1 nur kurze Zeit aktiv sein muß. In der ersten Quittung können bereits Daten zum Implantat 1 übertragen werden. Anhand des Inhalts und Typs der ersten Quittung wird angezeigt, ob im externen Gerät 2 weitere Daten zur Übertragung in Richtung lmplantat 1 anstehen.

Sobald das Implantat 1 mit der internen Verarbeitung der ersten Quittung der ersten Sendung fertig ist, wird eine weitere Sendung (wahlweise mit oder ohne Dateninhalt) ausgesandt. Die Quittung des externen Geräts auf diese erneute Sendung kann nun weitere Daten enthalten, die somit zum Implantat 1 übertragen werden. Wieder wird anhand des Inhalts und Typs der Quittung angezeigt, ob im externen Gerät 2 weitere Daten zur Übertragung in Richtung lmplantat 1 anstehen. Dieser Zyklus kann sich nun beliebig oft wiederholen, bis alle Daten in beide Richtungen übertragen worden sind und kein Gerät weitere Daten senden möchte.

Das Implantat sendet eine Nachricht zu einer beliebigen Zeit an die externe Gegenstelle. Ist mehr als eine Nachricht zu versenden, so werden sie nacheinander versendet. Die Quittungen werden jeweils daraufhin analysiert, ob die externe Gegenstelle 2 weitere Daten versenden will und es wird dann automatisch im Protokoll für die Übertragung von Daten in der Gegenrichtung (wie oben beschrieben) fortgefahren.

In einer weiteren Variante wird auch eine Reduktion des Energieverbrauchs auch im externen Gerät 2 erzielt. Sobald eine Nachricht vom externen Gerät 2 empfangen worden ist, synchronisieren sich Implantat und externes Gerät durch Verhandlung auf Zeitscheiben, wodurch periodische Zeiten definiert werden, in denen der Empfänger des externen Gerätes aktiv ist. Das Implantat 1 darf dann auch nur innerhalb dieser Zeitscheiben senden, um empfangen zu werden. Die obengenannten Handlungsanweisungen werden dementsprechend abgeändert, so daß das Implantat 1 nur innerhalb der Zeitscheiben senden kann und nicht mehr zu beliebigen Zeiten.

Für eine bestehende Kommunikation (Abfolge von Sendungen und Quittungen) gilt diese Einschränkung nicht, so daß der Empfänger im externen Gerät 2 nach dem Absetzen einer Quittung noch einige Zeit aktiv bleibt, um eine etwaige Folgesendung des Implantats 1 empfangen zu können. Das Implantat muß nicht in jeder Zeitscheibe auch eine Sendung absetzen. Wenn das externe Gerät 2 Kontakt zu mehreren Implantaten halten soll, so werden für jedes angemeldete Implantat eigene Zeitscheiben verwaltet, die sich nach Möglichkeit nicht überlappen sollten um Störungen bei gleichzeitigem Senden verschiedener Implantate zu vermeiden.

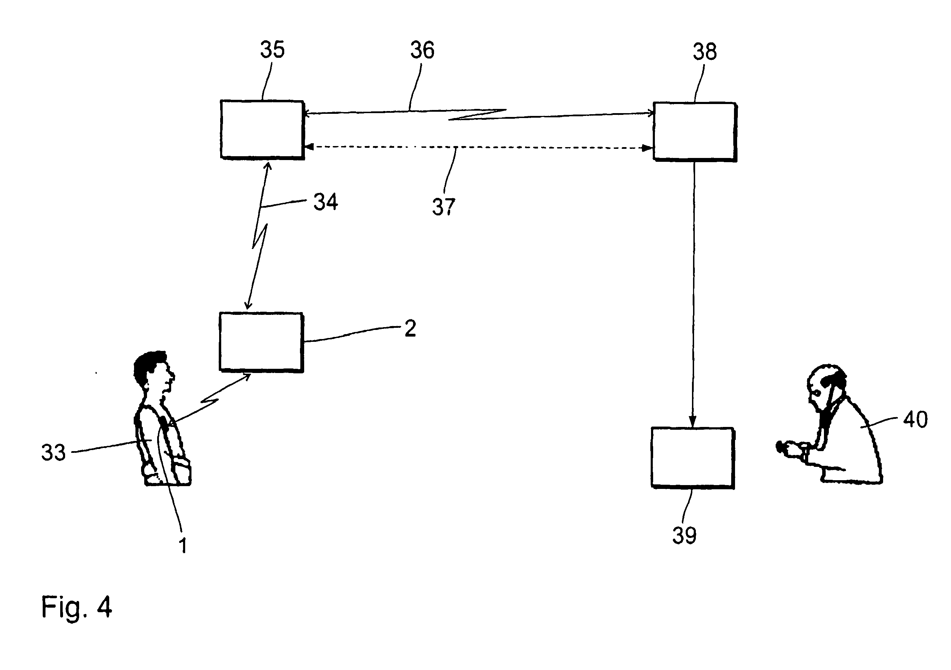

Die Zeitscheiben können im Rahmen der oben beschriebenen normalen bidirektionalen Kommunikation sowohl in der Dauer der aktiven als auch der passiven Phase als auch dem Synchronisationszeitpunkt verändert werden. Werden über einen bestimmten Zeitraum keine Sendungen mehr vom Implantat empfangen, so wird schrittweise die aktive Zeitscheibe auf Kosten der passiven verlängert, um ein Auseinanderdriften der Synchronität aufzufangen. Im Extremfall ist der Empfänger im externen Gerät dauernd aktiv. Dies ist auch der Reset-Zustand beim Einschalten. Figur 4 zeigt ein Blockschaltbild einer Variante eines Patientenüberwachungssystems unter Verwendung der vorliegenden Erfindung.

Dabei erfolgt eine Datenübertragung zwischen dem Implantat 1 und dem externen Gerät 2 aus Figur 1, das Implantat 1 wird dabei von einem Patienten 33 getragen. Von dem externen Gerät 2 werden die ausgelesenen Daten über eine Mobilfunkstrecke 34 eines Mobilfunknetzes an eine Mobilfunkstation 35 des Mobilfunknetzes übertragen und von dort ebenfalls über eine Mobilfunkstrecke 36 oder alternativ über eine Festnetzverbindung 37 an eine Servicezentrale 38 versandt, die als zentrale Speichereinrichtung und Überwachungseinrichtung dient.

Die Daten werden dabei als SMS versandt. Geht ein SMS bei der Servicezentrale 38 ein, so sendet diese einen Quittungs-SMS an das externe Gerät 2 zurück. Für die Mikroprozessoren in externen Geräten ist mit ihrer Real-Time-Clock nur die relative Zeit verfügbar. Die Zeit im Implantat 1 ist nicht justiert und nach einem halben Jahr auf +/-0,5 Stunden genau, so daß sie nicht verwendet werden kann. Wenn bei dem mobilen externen Gerät der Akku leer wird oder durch den Servicetechniker entnommen wird, kann es außerdem dazu kommen, daß die Real-Time-Clock völlig Zeit verliert. Das externe Gerät benötigt aber die aktuelle Zeit für die SMS-Nachricht, die an die Servicezentrale verschickt wird, um zu dokumentieren, wann das Ereignis vom Schrittmacher empfangen wurde.

Um die aktuelle Zeit im externen Gerät 2 einzustellen, d. h. eine Zeitsynchronisation des externen Geräts 2 durchzuführen, wird die Zeitsignatur (SMS-Header) des Quittungs-SMS verwendet, die Datum und Uhrzeit enthält.

Kommt diese Quittungs-SMS innerhalb von z.B. einer Minute zurück kann das externe Gerät 2 die absolute Zeit nach dem Header der Quittung einstellen, da gewährleistet ist, daß beide SMS weniger als eine Minute unterwegs waren. Ist zwischen gesendeter SMS und der Quittung eine längere Zeit vergangen, kann der SMS-Header der Quittung nicht ausgewertet werden, da nicht festgestellt werden kann, ob die Originalsendung oder die Quittung verzögert übertragen wurde. Die Zeit für eine SMS-Übertragung liegt erfahrungsgemäß im Bereich unter 1 Os, so daß man obiges Verfahren gut anwenden kann.

Da die Wahrscheinlichkeit sehr hoch ist, daß nach einem Power-On-Reset die Uhrzeit im externen Gerät 2 falsch oder zumindest weggelaufen ist, ist für diesen Fall folgendes vorgesehen. Wenn das externe Gerät 2 nach einem Power-On-Reset wieder ans Netz geht, meldet es dies sofort via SMS an die Servicezentrale 38. Mit der SMS-Quittung von der Servicezentrale 38 kann dann sofort die Uhr des externen Geräts gestellt werden.

Es kann festgelegt werden, daß sowohl die Servicezentrale 38, als auch das externe Gerät 2 mit der UTC-Zeit betrieben werden. Damit arbeitet das ganze System auf einer Zeit. Die Ortszeit wie z.B. MEZ oder MESZ muß dann bei Erstellung von Arztbriefen aus der UTC-Zeit ermittelt werden.

Sollte es bei der Ermittlung der absoluten Zeit Probleme geben, kann als Alternative noch die Zeitübertragung über das Modem 12 verwendet werden. Ein solcher Dienst wird in der Regel von verschiedenen Anbietern für das öffentliche Telefonnetz zur Verfügung gestellt.

Die zentrale Speichereinheit 38 ist mit einer vollautomatischen Datenbank ausgestattet. Die medizinischen Daten werden automatisch über TCP/IP-Verbindung in den Datenbank-Rechner eingelesen, dort werden sie entsprechend ihres Inhalts in die richtigen Tabellen eingetragen.

Die Datenbank speichert diese Daten und generiert entsprechend vordefinierter Regeln Informationen für den Arzt und den Systembetreuer und gibt diese über Fax, E-Mail, interne und externe Rechnerverbindung an die Zielgeräte 39 weiter. Aus Gründen von Kosten und Übersichtlichkeit werden die ausgegebenen Informationen zur späteren Sammelabfrage in der Datenbank aufbewahrt.

Die Zugriffsmöglichkeiten auf die Daten werden geregelt durch komplexe Rechteverteilung (z.B. Lesen, Schreiben, Verändern, Löschen, etc.) für verschiedene Benutzergruppen Datenverwalter, Klinik, Arzt.

Eine Zuordnung der eingehenden Daten zu Zeitpunkt, Implantat, Patient, externes Gerät und medizinischem Ansprechpartner (Arzt/Klinik) ist gewährleistet.

Bei Erreichen eines gewissen Füllegrades der Datenbank werden die Daten vom Rechner auf externe Medien (z.B. CD, Band) ausgelagert. Gemäß den medizinischen Richtlinien für Datensicherheit werden patientenbezogene Daten sofort (im Rahmen der Möglichkeiten) auf externe Read-Only-Medien (CD-ROM) gesichert. Ein geordneter Zugriff auf alle extern gesicherten Daten ist ständig über entsprechende Verweise in der Datenbank möglich.

Prioritätsgesteuerte Trigger in der Datenbank und ein angekoppeltes Expertensystem ermöglichen es, gezielt auf medizinische Sachverhalte zu reagieren: Es werden angepaßte Inhalte per Telefax, E-Mail, Telefon (zu Arzt oder Notrufzentrale) und Computerverbindung mit Arzt/Klinik verschickt. Der Arzt kann dabei einen zusammenfassenden oder verlorenen Report bezüglich des Patienten erneut anfordern. Er erhält medizinische Informationen in wenigen Minuten auch während der Untersuchung und zusammengefaßte Informationen zum nächsten Nachsorgetermin.

Das Übertragungsmedium und die Zieladressen sind dabei abhängig vom Auslösezeitpunkt konfigurierbar. Dadurch kann der betreuende Arzt 40 auf eine von ihm gewünschte Art und Weise Informationen über den Gesundheitszustand seines Patienten 33 erhalten, und der Patient 33 kann im Notfall ggf. schneller als üblich ärztliche Hilfe bekommen. Dieser Notfall wird sowohl durch objektiv gemessene medizinische Daten, als auch durch Tastendruck am Patientengerät 2 ausgelöst. Er erhält gegenüber den anderen Ereignissen höchste Priorität.

Während des Betriebs der Datenbank sind jederzeit manuelle Eingriffe und Abfragen durch Systembetreuer und andere Klienten (z.B. den Arzt 40) möglich.

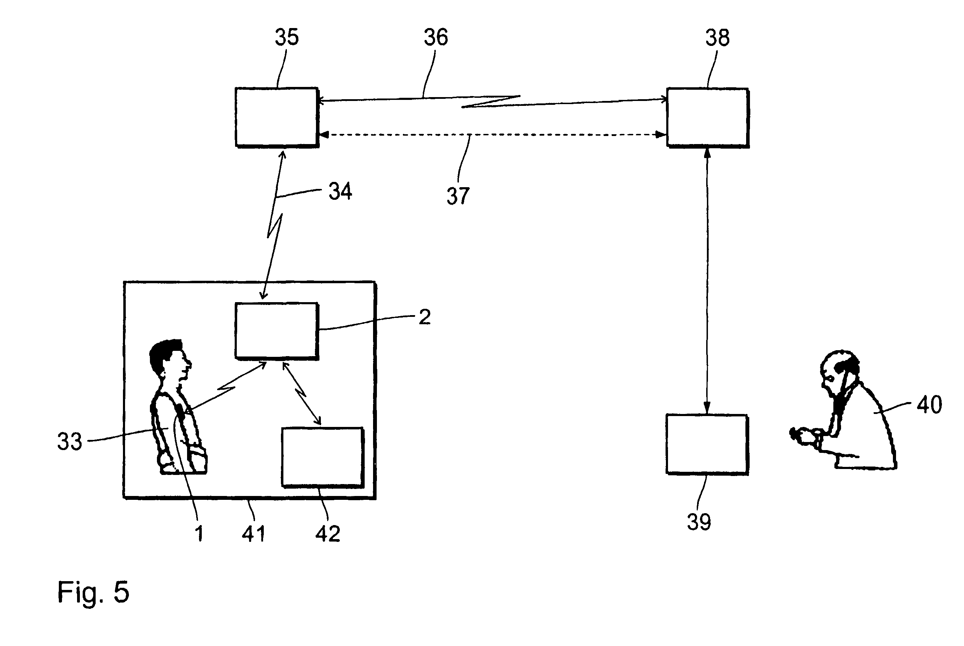

Figur 5 zeigt ein Blockschaltbild einer Variante eines Patientenüberwachungs- und - nachsorgesystems unter Verwendung der vorliegenden Erfindung.

Hierbei befindet sich der Patient 33 mit dem Implantat 1, und dem externen Gerät 2 zur Nachsorgeuntersuchung in einem Abfragebereich 41 einer Nachsorgeeinrichtung. Dabei wird, ausgelöst durch die mit dem externen Gerät bei betreten des Abfragebereichs 41 zusammenwirkende Auslöseeinrichtung automatisch eine Abfrage der ersten Daten aus dem Implantat 1 vor der Nachsorgeuntersuchung durch den Arzt 40 durchgeführt. Die Übertragung der ersten Daten erfolgt dabei mittels der langreichweitigen Telemetrieverbindung zwischen dem Implantat 1 und dem externen Gerät 2.

Das Ergebnis der Abfrage gelangt entsprechend aufbereitet über den zu Figur 4 beschriebenen Weg über die Servicezentrale 38 zum Zielgerät 39, von dem es an den Arzt 40 zu dessen Information ausgegeben wird. Gleichzeitig werden noch weitere patientenbezogene Daten aus der Datenbank der Servicezentrale 38 ausgegeben, so daß sich der Arzt 40 ein umfassendes Bild machen kann.

Die einzelne Untersuchung des Arztes (bei der er evtl. nur auf wenige Vergleichsdaten aus der Vergangenheit zurückgreift) wird durch ein Gesamtbild über die Zeit seit dem letzten Arztbesuch ergänzt.

Bei einer routinemäßigen Untersuchung erhält der Arzt ein Bild über mehrere Wochen oder Monate hinweg, was wesentlich aussagekräftiger ist als die momentane Untersuchung. Dabei werden Lebensgewohnheiten des Patienten gut abgebildet, wohin gegen gerade der Termin beim Arzt eine Ausnahmesituation vom gewohnten Leben darstellen kann (Fahrt zur Klinik bzw. zum Arzt, Treppensteigen, Streß im Verkehr etc.).

Bei der eigentlichen Untersuchung kann der Arzt (z.B. bei Belastungstests) auch sofort einen Report aus der Servicezentrale 38 anfordern, der dann nach wenigen Minuten zugestellt wird. Hierdurch können Parameteränderungen, die mit Hilfe des Programmers eingestellt wurden, sofort überprüft und ggf. noch optimiert werden.

Durch die Speicherung in der Datenbank und weitere Auswertemöglichkeiten durch das Expertensystem oder durch Vergleich mit anderen Fällen können weitere Reports erstellt werden. Durch Fortschritte in der medizinischen Erkenntnis können so neue Zusammenhänge erkannt werden. Diese werden dem Arzt dann beim nächsten Report mitgeteilt, ohne daß er bei neuen Erkenntnissen jeden seiner Patienten auf mögliche Auswirkungen überprüfen müßte.

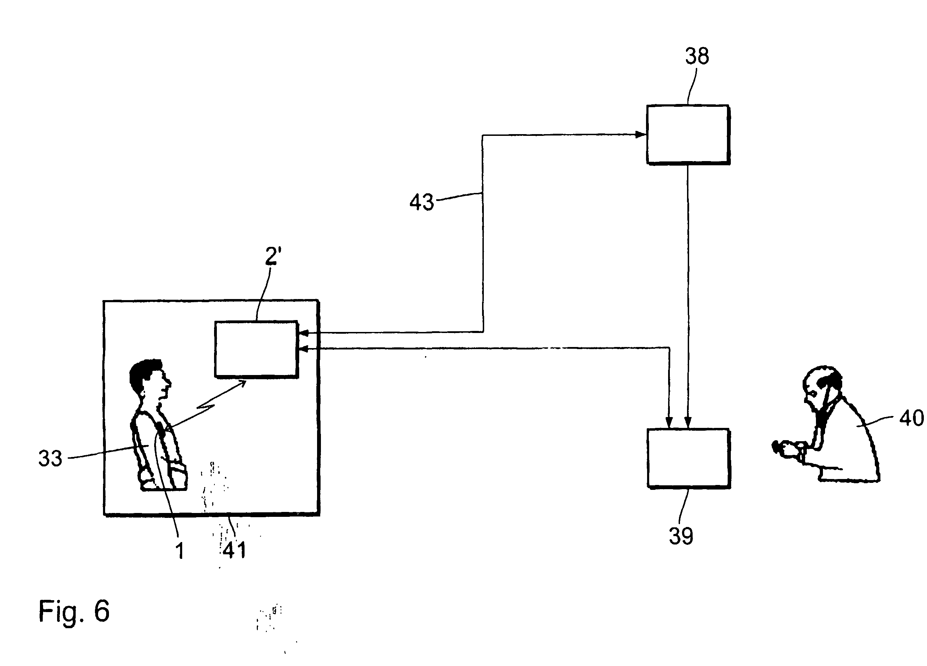

Figur 6 zeigt eine Variante der Ausführung aus Figur 5, bei der im Abfragebereich 41 der Nachsorgeeinrichtung ein externes Gerät 2' fest installiert ist. Hierbei wird die Abfrage ausgelöst, sobald sich der Patient 33 mit dem Implantat 1 für einen gewissen Zeitraum in Abfragereichweite des externen Geräts 2' befindet.

Das Ergebnis der Abfrage gelangt entsprechend aufbereitet direkt vom externen Gerät 2' zum Zielgerät 39, von dem es an den Arzt 40 zu dessen Information ausgegeben wird. Gleichzeitig werden über die Verbindung 43 noch weitere patientenbezogene Daten aus der Datenbank der Servicezentrale 38 abgefragt und am Zielgerät 39 ausgegeben, so daß sich der Arzt 40 auch hier ein umfassendes Bild machen kann.

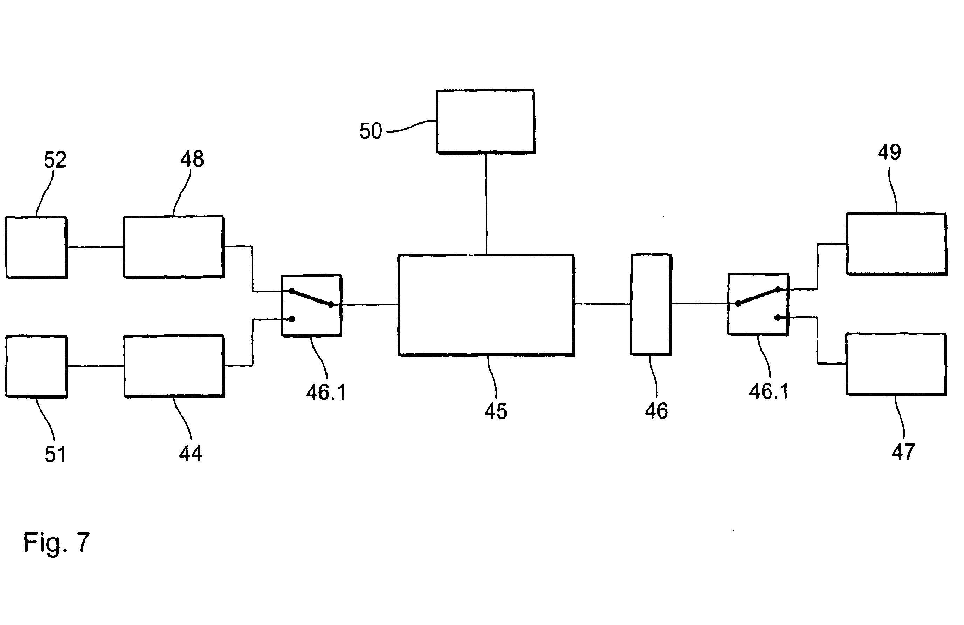

Figur 7 zeigt ein Blockschaltbild einer Telemetrieeinrichtung eines elektromedizinischen Implantats unter Verwendung der vorliegenden Erfindung.

Die Telemetrieeinrichtung weist einen Nahfeldsender 44, eine damit verbundenen Telemetrieeinheit 45 und eine mit der Telemetrieeinheit 45 verbundene Antennenschnittstelleneinrichtung 46 auf, über welche die erste Antenne 47 mit der Nahfeld-Sender/Empfängereinheit verbunden ist. Die übrigen notwendigen Systemkomponenten für eine Telemetrieeinrichtung, wie Zwischenspeicher, Ablaufsteuerung, Kodierung und Dekodierung, Treiber mit Schwellwertdetektor sind dabei in die Telemetrieeinheit 45 integriert.

Weiterhin ist zur Ausbildung einer Fernfeldtelemetrieeinrichtung ein mit der Telemetrieeinheit 45 verbundener Fernfeldsender 48 und eine mit der Antennenschnittstelleneinrichtung 46 verbundene Fernfeldantenne 49 vorgesehen.

Zum Umschalten zwischen Fernfeldsender 48 und Nahfeldsender 44 sind diese über eine Weiche 46.1 mit der Telemetrieeinheit 45 verbunden. Zum Umschalten zwischen erster Antenne 47 und Fernfeldantenne 49 sind diese über eine Weiche 46 mit der Antennenschnittstelleneinrichtung 45 verbunden. Es versteht sich jedoch, daß bei anderen Varianten die beiden Sender ebenso wie die beiden Antennen auch parallelgeschaltet an der Telemetrieeinheit 45 bzw. der Antennenschnittstelleneinrichtung 45 liegen können, da in der Regel Fern- und Nahfeldtelemetrie nicht gleichzeitig betrieben werden.

Zur Anpassung des Modulationsverfahrens für die jeweilige Telemetrie ist eine mit der Telemetrieeinheit 45 verbundene Anpassungslogikeinheit 50 vorgesehen. Die Fernfeldtelemetrieeinrichtung wird mit im wesentlichen derselben effektiven Datenrate wie die Nahfeldtelemetrieeinrichtung betrieben.

Weiterhin weisen die Nahfeldtelemetrieeinrichtung und die Fernfeldtelemetrieeinrichtung getrennte Energiespeicher 51 und 52 auf.

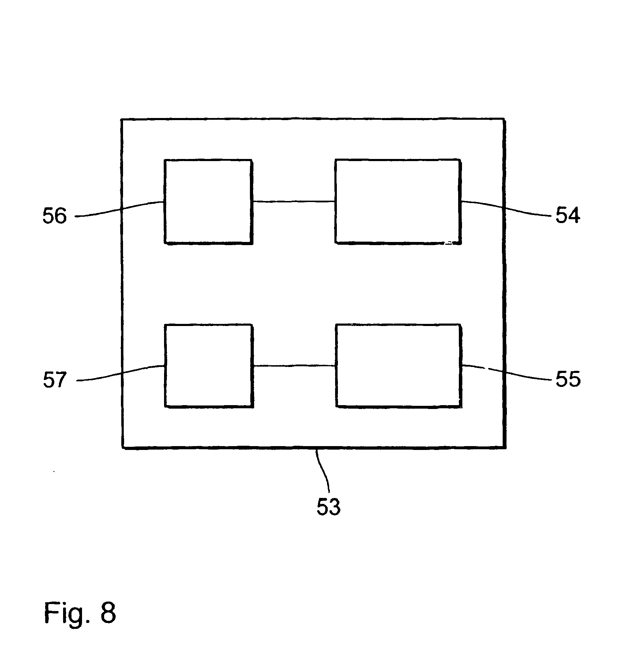

Figur 8 zeigt die Sender/Empfängereinheit einer Ausführung des Implantats 1, bei der für den Sender 54 und den Empfänger 55 getrennte Energiespeicher 56 und 57 vorgesehen sind. Dabei handelt es sich um separate Pufferkapazitäten 56 und 57. Diese Kapazitäten 56 und 57 müssen nur auf die für den jeweiligen Vorgang notwendige Spannung geladen werden. Der Energieverbrauch des einen Vorgangs beeinflußt nicht den Energievorrat für den anderen Vorgang. Die Vorgänge können somit unmittelbar aufeinander folgen, was für ein bidirektionales Kommunikationsprotokoll von Vorteil ist.

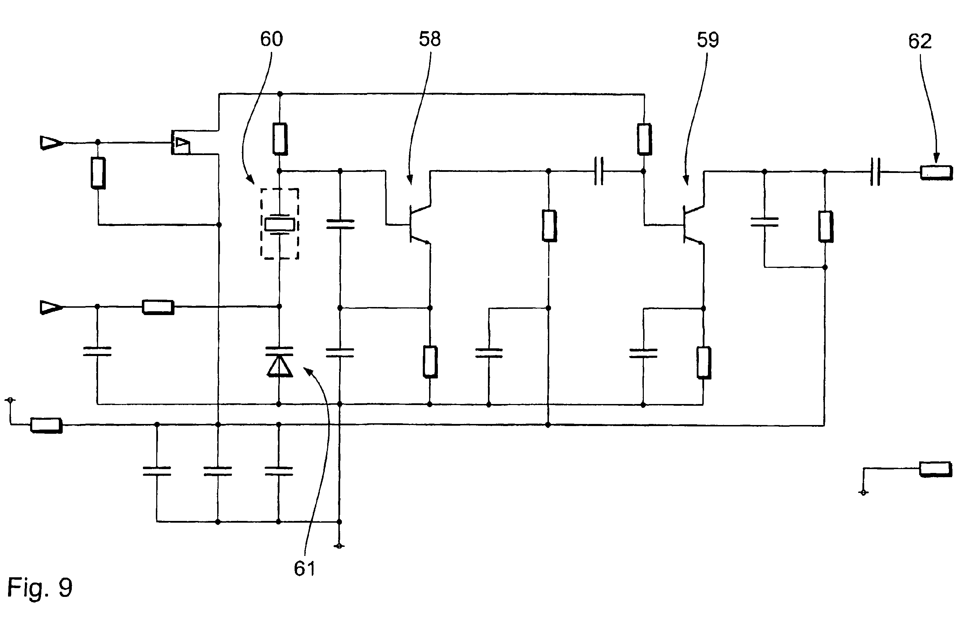

Figur 9 zeigt ein Schaltbild eines erfindungsgemäßen Senders der ersten Sender/Empfängereinheit einer Variante des Implantats 1 aus Figur 1.

Ein stabiler frequenzmodulierbarer Sender ist dabei mit zwei bipolaren Transistoren 58, 59 realisiert werden. Wobei der erste Transistor 58 in einer Colpitts- oder Clapp-Schaltung mit einem SAW-Resonator 60 einen SAW-stabilisierten Oszillator bildet und der zweite Transistor 59 als Pufferstufe und Antennentreiber dient.

Die Transistoren 58, 59 sind auf maximale Verstärkung bei geringstem Kollektorstrom ausgelegt. Mit einer Kapazitätsdiode 61 in Serie zum SAW-Resonator 60 kann die Frequenz moduliert werden. Der Frequenzhub, und damit die Datenrate und/oder die Reichweite des Senders, können jedoch dadurch erhöht werden, daß die Kapazitätsdiode durch eine PIN-Diode ersetzt wird, die von einem weiteren Transistor geschaltet wird.

Als Antenne kann eine einfache Drahtschleife oder ein offener Draht (Wurfantenne) in der Kontur des Headers des Implantats dienen. Insgesamt ist damit bei 400 MHz ein Stromverbrauch von weniger als 1 mA bei einer Reichweite von mehreren Metern möglich. Die Schaltung kann direkt aus einer von einer hochohmigen Batterie des Implantats 1 gespeisten Pufferkapazität oder einer niederohmigen Batterie versorgt werden. Eine Ladungspumpe ist nicht erforderlich.

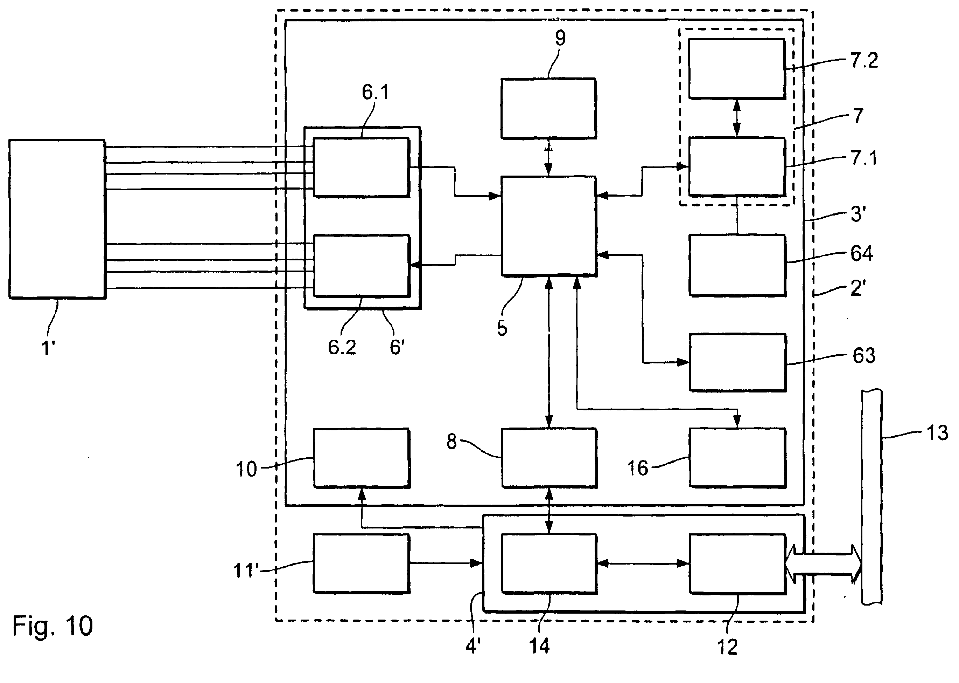

Figur 10 zeigt ein weiteres Ausführungsbeispiel einer erfindungsgemäßen Vorrichtung, die im wesentlichen derjenigen aus Figur 1 entspricht, weshalb hier lediglich auf die Unterschiede eingegangen werden soll.

Dabei handelt es sich um ein externes Gerät 2', das sich zur Überwachung, Ansteuerung und Übertragung von Daten aus verschiedensten elektronischen Geräten 1' eignet. Anwendungen können sein die Fernüberwachung von Anlagen, z.B. des Füllgrades von Getränke- oder Verkaufsautomaten, die Anbindung an die Haustechnik, z.B. Steuerung und Überwachung der Klimaanlage oder Heizung, die Kopplung an Alarmanlagen oder Glasbruchmeldeanlagen, z.B. Einbruchmeldung via Mobilfunk, die Kopplung an Meßsysteme, z.B. Wetterstationen oder Pegelmesser an Flüssen, die Kopplung an Systeme zur Verkehrsbeobachtung und -beeinflussung, z.B. Schilderbrücken etc. Weiterhin kann es als Abhöranlage oder als Mobiltelefon mit digitalen Sonderfunktionen eingesetzt werden.

Die Unterschiede zur Ausführung aus Figur 1 betreffen im wesentlichen das Mobilteil 3' des externen Geräts 2'. Die erste Schnittstelleneinrichtung umfaßt dabei 4 potentialfreie Eingänge 6.1 über Optokoppler und 4 potentialfreie Ausgänge 6.2. Dank der Potentialtrennung der Ein- und Ausgänge 6.1 und 6.2 eignet sich das externe Gerät 2' für die Überwachung und Steuerung von digitalen Zuständen.

Weiterhin entfällt der Empfangs- und Sendeteil 6 aus Figur 1, statt dessen wird die freiwerdende serielle Schnittstelle 63 (RS 232) über einen 9-poligen Stecker herausgeführt. Bei der RS 232 genügt ein Softwarehandshake mit drei Leitungen (RXD, TXD, OND). Damit ist die Übertragungsrate ebenfalls auf 9600 Baud begrenzt. Die serielle Schnittstelle 63 ist nicht potentialfrei ausgelegt. Über einem weiteren Stecker 64 können die Verbindungen des Mobilfunkmoduls 7.1 zu einem Mikrofon, einem Lautsprecher und optional einem Buzzer geführt werden (6 Leitungen), so daß für weitere Einsatzgebiete kann eine Hör/Sprechkombination und ein Buzzer hinzugefügt werden können.

Die Eingänge 6.1 liegen im Bereich 1 bei -3V bis 1,5V L-Pegel, 3,5 bis 8V H-Pegel und 10 mA Eingangsstrom; im Bereich 2 bei -3V bis 8V L-Pegel, 18V bis 30V H-Pegel und 10 mA Eingangsstrom. Die Zustandserkennung erfolgt über eine LED-Anzeige bei maximaler Schaltfrequenz 100Hz Rechteck und einer Trennspannung von 2,5kV.

Die Ausgänge weisen 15 bis 30V DC separate Spannungseinspeisung, einen Laststrom von 200mA, Kurzschlußschutz, Schutz gegen thermische Überlastung und eine Trennspannung von 2,5kV auf. Die Potentialtrennung wird über Optokoppler, die Leistungs-Schaltfunktion über MOS-FETs realisiert.

Ein Unterschied zur Ausführung aus Figur 1 bezüglich der Basisstation 4' besteht lediglich hinsichtlich der Stromversorgung durch ein Steckernetzteil 11' statt dem Netzteil in der Basisstation. Eine weitere Ausführungsmöglichkeit besteht darin, daß die Basisstation wegfällt und das externe Gerät direkt über ein Netzteil versorgt wird. Bei Netzausfall arbeitet das Mobilteil dann noch etwa 20 Stunden weiter.

Die Erfindung beschränkt sich in ihrer Ausführung nicht auf die vorstehend angegebenen bevorzugten Ausführungsbeispiele. Vielmehr ist eine Anzahl von Varianten möglich, welche von der dargestellten Lösung auch bei grundsätzlich anders gearteten Ausführungen Gebrauch macht.

## Patentansprüche

1. Verfahren zur Datenübertragung zwischen
einem elektromedizinischen Implantat (1) mit einer ersten Sender/Empfängereinheit, insbesondere einem Implantat für kardiologische Anwendungen,
und
einem zugeordneten externen Gerät (2) mit einer zweiten Sender/Empfängereinheit (6),
bei dem die Datenübertragung mit einem von der einen Sende/Empfängereinheit an die andere Sender/Empfängereinheit gesendeten Auslösesignal beginnt, das im Normalbetrieb des Implantats (1) in vorgebbaren ersten Zeitintervallen gesendet wird,
wobei das Auslösesignal stets von der ersten Sender/Empfängereinheit ausgesendet und wenigstens die Empfangsbereitschaft der ersten Sender/Empfängereinheit nach Aussenden des Auslösesignal für ein zweites Zeitintervall aufrechterhalten wird,
**dadurch gekennzeichnet,**
**dass** die zweite Sender/Empfängereinheit (6) auf eine Übertragung von Daten durch die erste Sender/Empfängereinheit hin eine erste Quittung an die erste Sender/Empfängereinheit sendet, wobei die erste Quittung wenigstens eine erste Steuerinformation zur Steuerung der Empfangsbereitschaft der ersten Sender/Empfängereinheit enthält.

2. Verfahren nach Anspruch 1, **dadurch gekennzeichnet, dass** wenigsten der Empfangsteil der ersten Sender/Empfängereinheit nach Beendigung des zweiten Zeitintervalls während einer Ruhephase, die bis zum nächsten Auslösesignal reicht, ausgeschaltet wird.

3. Verfahren nach Anspruch 1 oder 2, **dadurch gekennzeichnet, dass** das zweite Zeitintervall kürzer ist als das erste Zeitintervall.

4. Verfahren nach einem der vorstehenden Ansprüche, **dadurch gekennzeichnet, dass** das Auslösesignal von einem zu übertragenden ersten Datensatz gebildet ist oder diesen enthält.

5. Verfahren nach einem der vorstehenden Ansprüche, **dadurch gekennzeichnet, dass** die erste Quittung von einem zu übertragenden zweiten Datensatz gebildet ist oder diesen enthält.

6. Verfahren nach einem der vorstehenden Ansprüche, **dadurch gekennzeichnet, dass** das externe Gerät (2) eine erste Plausibilitätsprüfung der von der ersten Sende/Empfängereinheit übertragenen Daten durchführt und die erste Quittung in Abhängigkeit von der Plausibilität der übertragenen Daten eine zweite Steuerinformation zur Steuerung der ersten Sende/Empfängereinheit enthält, wobei die zweite Steuerinformation bei mangelnder Plausibilität der übertragenen Daten, ein erstes Steuersignal zum Auslösen einer erneuten Übertragung von Daten durch die erste Sende/Empfängereinheit enthält.

7. Verfahren nach Anspruch 5, **dadurch gekennzeichnet, dass** die erste Sende/Empfängereinheit auf das erste Steuersignal eine erneute Übertragung von Daten nur durchführt, sofern eine zur Vermeidung einer Überbeanspruchung der Energieversorgung des Implantats (1) ausreichend geringe Anzahl erneuter Übertragungen nicht überschritten ist.

8. Verfahren nach Anspruch 6 oder 7, **dadurch gekennzeichnet, dass** die zweite Sende/Empfängereinheit (6) zur Kontrolle der Übertragung der Daten durch das Implantat (1) bei vorhandener Plausibilität der übertragenen Daten wenigstens einen Teil der übertragenen Daten an die erste Sende/Empfängereinheit sendet.

9. Verfahren nach Anspruch 8, **dadurch gekennzeichnet, dass** das Implantat (1) nach der Kontrolle der Übertragung der Daten über die erste Sende/Empfängereinheit eine zweite Quittung an die zweite Sende/Empfängereinheit (2) sendet, wobei die zweite Quittung bei Feststellung einer erfolgreichen Übertragung der Daten eine die Gültigkeit der Übertragung repräsentierende erste Signatur enthält, und das Implantat (1) wenigstens die Empfangsbereitschaft der ersten Sende/Empfängereinheit abstellt.

10. Verfahren nach Anspruch 9, **dadurch gekennzeichnet, dass** das externe Gerät (2) eine zweite Plausibilitätsprüfung der zweiten Quittung durchführt und bei festgestellter mangelnder Plausibilität der zweiten Quittung nach Ablauf eines weiteren Zeitintervalls nach Absendung der zweiten Quittung eine Abfrage des Implantats (1) durchführt, wobei das Implantat (1) nach Ablauf des weiteren Zeitintervalls die Empfangs- und Sendebereitschaft der ersten Sende/Empfängereinheit für ein erneutes weiteres Zeitintervall aufnimmt das ausreicht, um eine Anfrage vom externen Gerät (2) aufzunehmen und zu beantworten, und wobei die Beantwortung der Anfrage durch erneutes Senden der zweiten Quittung und/oder der zuletzt gesandten Daten erfolgt.

11. Verfahren nach einem der vorstehenden Ansprüche, **dadurch gekennzeichnet, dass** bei Feststellung einer fehlerhaften Übertragung von Daten eine erneute Übertragung von Daten durch die erste Sende/Empfängereinheit erfolgt, sofern eine zur Vermeidung einer Überbeanspruchung der Energieversorgung des Implantats (1) ausreichend geringe Anzahl erneuter Übertragungen nicht überschritten ist.

12. Verfahren nach einem der vorstehenden Ansprüche, **dadurch gekennzeichnet, dass** die erneute Übertragung nach Ablauf eines Wartezeitintervalls erfolgt, wobei bei mehrfacher erneuter Übertragung die Länge des Wartezeitintervalls vorzugsweise ansteigt.

13. Verfahren nach einem der vorstehenden Ansprüche, **dadurch gekennzeichnet, dass** nach erneuter Übertragung von Daten durch die erste Sende/Empfängereinheit die Verfahrensschritte beginnend mit der Plausibilitätsprüfung erneut durchlaufen werden.

14. Verfahren nach einem der vorhergehenden Ansprüche, **dadurch gekennzeichnet, dass** die zweite Sende/Empfängereinheit (6) im Ausgangszustand bis zum ersten Datenaustausch mit dem Implantat (1) im wesentlichen permanent empfangsbereit ist und wenigstens während des ersten Datenaustauschs die Sende- bzw. Empfangsbereitschaft der zweiten Sende/Empfängereinheit (6) auf ein periodisches Sende- bzw. Empfangsbereitschaftsintervall reduziert, wobei die zweite Sende/Empfängereinheit (6) mit der ersten Sende/Empfängereinheit derart synchronisiert wird, dass sich die Sende- bzw. Empfangsbereitschaftsintervalle der ersten und zweiten Sende/Empfängereinheit überschneiden.

15. Verfahren nach Anspruch 14, **dadurch gekennzeichnet, dass** im Falle des Nichteintreffens von Übertragungen der ersten Sende/Empfängereinheit bei der zweiten Sende/Empfängereinheit (6) über eine vorgegebene Anzahl von Sende- bzw. Empfangsbereitschaftsintervallen der zweiten Sende/Empfängereinheit (6) das Sende- bzw. Empfangsbereitschaftsintervall der zweiten Sende/Empfängereinheit (6) zum Auffangen eines Auseinanderdriftens der Synchronität verlängert wird.

16. Verfahren nach einem der vorhergehenden Ansprüche, **dadurch gekennzeichnet, dass** wenigstens das erste Zeitintervall während des Betriebs durch Übersenden einer zweiten Steuerinformation durch die zweite Sende/Empfängereinheit (6) an die empfangsbereite erste Sende/Empfängereinheit veränderbar ist.

17. Verfahren nach einem der vorhergehenden Ansprüche, **dadurch gekennzeichnet, dass** das erste Zeitintervall in Abhängigkeit von den Betriebsparametern des Implantats (1) verändert wird.

18. Verfahren nach einem der vorhergehenden Ansprüche, **dadurch gekennzeichnet, dass** die erste Sende/Empfängereinheit bei Vorliegen entsprechender Betriebsparameter des Implantats (1) zur Auslösung eines Alarmsignals ein Notfall-Auslösesignal an die zweite Sende/Empfängereinheit (6) aussendet.

19. Vorrichtung zur Datenübertragung zwischen einem elektromedizinischen Implantat (1) mit einer ersten Sender/Empfängereinheit, insbesondere einem Implantat für kardiologische Anwendungen, und einem zugeordneten externen Gerät (2) mit einer zweiten Sender/Empfängereinheit (6), bei der die Datenübertragung mit einem Auslösesignal beginnt, welches von der einen Sende/Empfängereinheit an die andere Sender/Empfängereinheit im Normalbetrieb des Implantats (1) in vorgebbaren ersten Zeitintervallen gesendet wird,wobei das Auslösesignal stets von der ersten Sender/ Empfängereinheit ausgesendet und wenigstens die Empfangsbereitschaft der ersten Sender/Empfängereinheit nach Aussenden des Auslösesignals für ein zweites Zeitintervall aufrechterhalten wird, **dadurch gekennzeichnet, dass** die Vorrichtung derart ausgebildet ist, dass die zweite Sender/Empfängereinheit (6) auf eine Übertragung von Daten durch die erste Sender/Empfängereinheit hin eine erste Quittung an die erste Sender/Empfängereinheit sendet, wobei die erste Quittung wenigstens eine erste Steuerinformation zur Steuerung der Empfangsbereitschaft der ersten Sender/Empfängereinheit enthält.

20. Vorrichtung nach Anspruch 19, **dadurch gekennzeichnet, dass** wenigstens der Empfangsteil der ersten Sender/Empfängereinheit nach Beendigung des zweiten Zeitintervalls während einer Ruhephase, die bis zum nächsten Auslösesignal reicht, ausgeschaltet bleibt.

## Claims

1. A method for data transmission between
an electromedical implant (1) having a first transceiver unit, particularly an implant for cardiological applications,
and
an associated external device (2) having a second transceiver unit (6),
in which the data transmission begins with a triggering signal transmitted from one transceiver unit to the other transceiver unit, which is transmitted at predefinable first time intervals in normal operation of the implant (1),
the triggering signal always being transmitted from the first transceiver unit and at least the receive readiness of the first transceiver unit being maintained for a second time interval after transmitting the triggering signal,
**characterized in that**
the second transceiver unit (6) transmits a first acknowledgment to the first transceiver unit for a transmission of data by the first transceiver unit, the first acknowledgment containing at least first control information for controlling the receive readiness of the first transceiver unit.

2. The method according to Claim 1,
**characterized in that** at least the receiver part of the first transceiver unit is turned off during a rest phase, which extends until the next triggering signal, after ending the second time interval.

3. The method according to Claim 1 or 2,
**characterized in that** the second time interval is shorter than the first time interval.

4. The method according to one of the preceding claims,
**characterized in that** the triggering signal is formed by a first dataset to be transmitted or contains this dataset.

5. The method according to one of the preceding claims,
**characterized in that** the first acknowledgment is formed by a second dataset to be transmitted or contains this dataset.

6. The method according to one of the preceding claims,
**characterized in that** the external device (2) performs a first plausibility check of the data transmitted by the first transceiver unit and the first acknowledgment contains second control information for controlling the first transceiver unit as a function of the plausibility of the transmitted data, the second control information, in the event of a lack of plausibility of the transmitted data, containing a first control signal for triggering renewed transmission of data by the first transceiver unit.

7. The method according to Claim 5,
**characterized in that** the first transceiver unit only performs a renewed transmission of data upon the first control signal if a number of renewed transmissions which is sufficiently low to avoid overtaxing the power supply of the implant (1) is not exceeded.

8. The method according to Claim 6 or 7,
**characterized in that** the second transceiver unit (6) transmits at least a part of the transmitted data to the first transceiver unit to check the transmission of the data by the implant (1) if transmitted data is plausible.

9. The method according to Claim 8,
**characterized in that** the implant (1) transmits a second acknowledgment to the second transceiver unit (2) after the check of the transmission of the data via the first transceiver unit, the second acknowledgment containing a first signature representing the validity of the transmission if a successful transmission of the data has been established, and the implant (1) stops at least the receive readiness of the first transceiver unit.

10. The method according to Claim 9,
**characterized in that** the external device (2) performs a second plausibility check of the second acknowledgment and, if a lack of plausibility of the second acknowledgment is established, performs a query of the implant (1) after expiration of a further time interval after transmission of the second acknowledgment, the implant (1), after expiration of the further time interval, accepting the receive and transmit readiness of the first transceiver unit for a renewed further time interval which is sufficient to accept and respond to a query from the external device (2), and the response to the query being performed through renewed transmission of the second acknowledgment and/or the last transmitted data.

11. The method according to one of the preceding claims,
**characterized in that**, if a faulty transmission of data is established, a renewed transmission of data is performed by the first transceiver unit if a number of renewed transmissions which is sufficiently low to avoid overtaxing the power supply of the implant (1) has not been exceeded.

12. The method according to one of the preceding claims,
**characterized in that** the renewed transmission is performed after expiration of a waiting time interval, the length of the waiting time interval preferably increasing in the event of multiple renewed transmissions.

13. The method according to one of the preceding claims,
**characterized in that**, after renewed transmission of data by the first transceiver unit, the method steps are executed again beginning with the plausibility check.

14. The method according to one of the preceding claims,
**characterized in that** the second transceiver unit (6) is essentially permanently ready to receive in the starting state up to the first data exchange with the implant (1) and reduces the transmit and/or receive readiness of the second transceiver unit (6) to a periodic transmit and/or receive readiness interval at least during the first data exchange, the second transceiver unit (6) being synchronized with the first transceiver unit in such a way that the transmit and/or receive readiness intervals of the first and second transceiver unit overlap.

15. The method according to Claim 14,
**characterized in that** if transmissions of the first transceiver unit are not received by the second transceiver unit (6) over a predefined number of transmit and/or receive readiness intervals of the second transceiver unit (6), the transmit and/or receive readiness interval of the second transceiver unit (6) is lengthened to pick up drifting apart of the synchronism.

16. The method according to one of the preceding claims,
**characterized in that** at least the first time interval is changeable during operation through transmission of second control information by the second transceiver unit (6) to the first transceiver unit, which is ready to receive.

17. The method according to one of the preceding claims,
**characterized in that** the first time interval is changed as a function of the operating parameters of the implant (1).

18. The method according to one of the preceding claims,
**characterized in that** the first transceiver unit transmits an emergency triggering signal to the second transceiver unit (6) if corresponding operating parameters of the implant (1) for triggering an alarm signal exist.

19. A device for data transmission between an electromedical implant (1) having a first transceiver unit, in particular an implant for cardiological applications, and an associated external device (2) having a second transceiver unit (6), in which the data transmission begins with a triggering signal which is transmitted by one transceiver unit to the other transceiver unit in normal operation of the implant (1) at predefinable first time intervals, the triggering signal always being transmitted by the first transceiver unit and at least the receive readiness of the first transceiver unit being maintained for a second time interval after transmitting the triggering signal,
**characterized in that** the device is implemented in such a way that the second transceiver unit (6) transmits a first acknowledgment to the first transceiver unit after a transmission of data by the first transceiver unit, the first acknowledgment containing at least first control information for controlling the receive readiness of the first transceiver unit.

20. The device according to Claim 19,
**characterized in that** at least the receiver part of the first transceiver unit remains turned off after ending the second time interval during a rest phase, which extends until the next triggering signal.

## Revendications

1. Procédé pour la transmission de données entre
un implant (1) électromédical avec une première unité émetteur/récepteur, en particulier un implant pour les applications cardiologiques,
et
un appareil (2) externe affecté avec une seconde unité émetteur/récepteur (6), par lequel la transmission de données commence avec un signal de déclenchement envoyé par la une unité émetteur/récepteur à l'autre unité émetteur/récepteur, qui est envoyé avec le fonctionnement normal de l'implant (1) dans des premiers intervalles de temps prédéfinissables,
par lequel le signal de déclenchement est toujours envoyé par la première unité émetteur/récepteur et au moins la disponibilité de réception de la première unité émetteur/récepteur est maintenue pour un second intervalle de temps après l'émission du signal de déclenchement,
**caractérisé en ce que**
la seconde unité émetteur/récepteur (6) envoie un premier accusé de réception à la première unité émetteur/récepteur, suite à une transmission de données par la première unité émetteur/récepteur, le premier accusé de réception contenant au moins une première information de commande pour la commande de la disponibilité de réception de la première unité émetteur/récepteur.

2. Procédé selon la revendication 1, **caractérisé en ce que** au moins la partie de réception de la première unité émetteur/récepteur est déconnectée après la fin du second intervalle de temps pendant une phase de repos qui va jusqu'au prochain signal de déclenchement.

3. Procédé selon la revendication 1 ou 2, **caractérisé en ce que** le second intervalle de temps est plus court que le premier intervalle de temps.

4. Procédé selon l'une quelconque des revendications précédentes, **caractérisé en ce que** le signal de déclenchement est formé par un premier ensemble de données à transmettre ou contient celui-ci.

5. Procédé selon l'une quelconque des revendications précédentes, **caractérisé en ce que** le premier accusé de réception est formé par un second ensemble de données à transmettre ou contient celui-ci.

6. Procédé selon l'une quelconque des revendications précédentes, **caractérisé en ce que** l'appareil (2) externe effectue un premier contrôle de vraisemblance des données transmises par la première unité émetteur/récepteur et le premier accusé de réception contient en fonction de la vraisemblance des données transmises une seconde information de commande pour la commande de la première unité émetteur/récepteur, la seconde information de commande contenant en cas d'absence de vraisemblance des données transmises un premier signal de commande pour le déclenchement d'une nouvelle transmission de données par la première unité émetteur/récepteur.

7. Procédé selon la revendication 5, **caractérisé en ce que** la première unité émetteur/récepteur effectue à la suite du premier signal de commande une nouvelle transmission de données seulement si un certain nombre de nouvelles transmissions, qui est suffisamment faible pour éviter une sursollicitation de l'alimentation en énergie de l'implant (1), n'est pas dépassé.

8. Procédé selon la revendication 6 ou 7, **caractérisé en ce que** la seconde unité émetteur/récepteur (6) envoie pour le contrôle de la transmission des données par l'implant (1), en cas de vraisemblance des données transmises, au moins une partie des données transmises à la première unité émetteur/récepteur.

9. Procédé selon la revendication 8, **caractérisé en ce que** l'implant (1) envoie après le contrôle de la transmission des données par la première unité émetteur/récepteur un second accusé de réception à la seconde unité émetteur/récepteur (2), le second accusé de réception contenant une première signature représentant la validité de la transmission en cas de constatation d'une transmission réussie des données et l'implant (1) arrête au moins la disponibilité de réception de la première unité émetteur/récepteur.

10. Procédé selon la revendication 9, **caractérisé en ce que** l'appareil (2) externe effectue un premier contrôle de vraisemblance du second accusé de réception, et, si l'on constate l'absence de vraisemblance du second accusé de réception, effectue une interrogation de l'implant (1) après l'écoulement d'un nouvel intervalle de temps suivant l'envoi du second accusé de réception, l'implant (1) recevant après l'écoulement du nouvel intervalle de temps la disponibilité de réception et d'émission de la première unité émetteur/récepteur pour un autre intervalle de temps nouveau qui suffit pour recevoir une demande de l'appareil (2) externe et d'y répondre, et la réponse à la demande s'effectuant par un nouvel envoi du second accusé de réception et/ou des dernières données envoyées.

11. Procédé selon l'une quelconque des revendications précédentes, **caractérisé en ce que**, si l'on constate une transmission erronée de données, une nouvelle transmission de données est effectuée par la première unité émetteur/récepteur dans la mesure où un nombre suffisamment faible de nouvelles transmissions pour éviter une sursollicitation de l'alimentation en énergie de l'implant (1) n'est pas dépassé.

12. Procédé selon l'une quelconque des revendications précédentes, **caractérisé en ce que** la nouvelle transmission intervient après l'écoulement d'un intervalle de délai d'attente, la longueur de l'intervalle d'attente augmentant de préférence en cas de nouvelle transmission multiple.

13. Procédé selon l'une quelconque des revendications précédentes, **caractérisé en ce que**, après la nouvelle transmission des données par la première unité émetteur/récepteur, les étapes du procédé sont réalisées de nouveau en commençant avec le contrôle de vraisemblance.

14. Procédé selon l'une quelconque des revendications précédentes, **caractérisé en ce que** la seconde unité émetteur/récepteur (6) est prête pour la réception sensiblement en permanence dans l'état initial jusqu'au premier échange de données avec l'implant (1) et réduit au moins pendant le premier échange de données la disponibilité d'émission et de réception de la seconde unité émetteur/récepteur (6) à un intervalle périodique de disponibilité d'émission ou de réception, la seconde unité émetteur/récepteur (6) étant synchronisée avec la première unité émetteur/récepteur de telle sorte que les intervalles de disponibilité d'émission ou de réception de la première et de la seconde unité émetteur/récepteur se recoupent.

15. Procédé selon la revendication 14, **caractérisé en ce que**, dans le cas de non-arrivée de transmissions de la première unité émetteur/récepteur sur la seconde unité émetteur/récepteur (6), l'intervalle de disponibilité d'émission ou de réception de la seconde unité émetteur/récepteur (6) est prolongée au moyen d'un nombre prédéfini d'intervalles de disponibilité d'émission ou de réception de la seconde unité émetteur/récepteur (6) pour recueillir un écartement du synchronisme.

16. Procédé selon l'une quelconque des revendications précédentes, **caractérisé en ce qu'**au moins le premier intervalle de temps peut être modifié pendant l'exploitation par la transmission d'une seconde information de commande par la seconde unité d'émission/réception (6) à la première unité émetteur/récepteur prête pour la réception.

17. Procédé selon l'une quelconque des revendications précédentes, **caractérisé en ce que** le premier intervalle de temps est modifié en fonction des paramètres de service de l'implant (1).

18. Procédé selon l'une quelconque des revendications précédentes, **caractérisé en ce que** la première unité émetteur/récepteur envoie un signal de déclenchement d'urgence à la seconde unité émetteur/récepteur (6) pour le déclenchement d'un signal d'alarme en cas de présence de paramètres de service correspondants.

19. Dispositif pour la transmission de données entre un implant (1) électromédical avec une unité émetteur/récepteur, en particulier un implant pour les applications cardiologiques, et un appareil (2) externe attribué avec une seconde unité émetteur/récepteur (6), sur laquelle la transmission de données commence avec un signal de déclenchement qui est envoyé par une première unité émetteur/récepteur à l'autre unité émetteur/récepteur pendant l'exploitation normale de l'implant (1) à des premiers intervalles de temps prédéfinissables, le signal de déclenchement étant toujours envoyé par la première unité émetteur/récepteur et au moins la disponibilité de réception de la première unité émetteur/récepteur étant maintenue pour un second intervalle de temps après l'envoi du signal de déclenchement, **caractérisé en ce que** le dispositif est conçu de telle sorte que
la seconde unité émetteur/récepteur (6) envoie suite à une transmission de données par la première unité émetteur/récepteur un premier accusé de réception à la première unité émetteur/récepteur, le premier accusé de réception contenant au moins une première information de commande pour la commande de la disponibilité de réception de la première unité émetteur/récepteur.

20. Dispositif selon la revendication 19, **caractérisé en ce qu'**au moins la partie de réception de la première unité émetteur/récepteur reste déclenchée après la fin du seconde intervalle de temps pendant une phase de repos qui va jusqu'au prochain signal de déclenchement.
